(19) Europäisches Patentamt
European Patent Office
Office européen des brevets

(11) Veröffentlichungsnummer: **0 037 465**
**A2**

# (12) EUROPÄISCHE PATENTANMELDUNG

(21) Anmeldenummer: 81101712.8

(22) Anmeldetag: 09.03.81

(51) Int. Cl.³: C 07 C 107/00
C 09 B 29/00
//C09B31/00, C09B35/04

(30) Priorität: 15.03.80 DE 3010104

(43) Veröffentlichungstag der Anmeldung:
14.10.81 Patentblatt 81/41

(84) Benannte Vertragsstaaten:
BE CH DE FR GB IT LI NL

(71) Anmelder: BASF Aktiengesellschaft
Carl-Bosch-Strasse 38
D-6700 Ludwigshafen(DE)

(72) Erfinder: Kurtz, Walter, Dr.
Sonnenwendstrasse 73
D-6702 Bad Duerkheim(DE)

(72) Erfinder: Lamm, Gunther, Dr.
Heinrich-Heine-Strasse 7
D-6733 Hassloch(DE)

## (54) Aminoazoverbindungen.

(57) Die Erfindung betrifft Aminoazoverbindungen der allgemeinen Formel

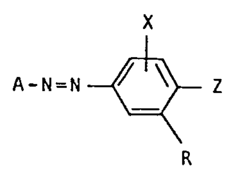

in der
A der Rest einer Diazo- oder Kupplungskomponente und
R ein Rest der Formel
CN, COOR¹, COSR¹, CONH₂, CONHNH₂, CONH-NH-CO R¹,

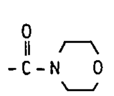 , 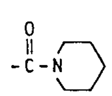 , 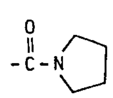 ,

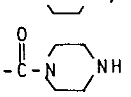 , 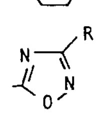 ,

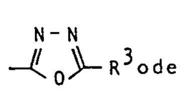 oder 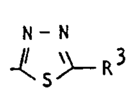

Z Amino,
R und Z zusammen ein Rest der Formel -NHCO-O-CO-,
X Wasserstoff, Chlor, Brom, Methoxy, Äthoxy, Propoxy, $SO_3H$, Butoxy, Phenoxy, Methyl, Äthyl, Propyl, Butyl, Acetylamino, Dimethylamino, Diäthylamino, Carboxyl oder $-NO_2$ sind, wobei
$R^1$ gegebenenfalls substituiertes Alkyl, Alkenyl, Cycloalkyl oder Aryl,
$R^2$ Wasserstoff oder gegebenenfalls substituiertes Alkyl und
$R^3$ Wasserstoff oder einen Rest $R^1$ bedeuten und wobei Aminobenzoesäureamide, Mono- und Dichloraniline als Diazokomponenten A ausgeschlossen sind, wenn $R^1$ $C_1$-bis $C_4$-Alkyl, Methoxyäthyl oder Butoxyäthoxyäthyl ist.

Die erfindungsgemäßen Verbindungen eignen sich vorzüglich als Diazokomponenten und Zwischenprodukte zur Herstellung von Farbstoffen.

EP 0 037 465 A2

BASF Aktiengesellschaft O. Z. 0050/034355

## Aminoazoverbindungen

Die Erfindung betrifft Verbindungen der allgemeinen Formel I

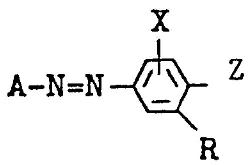

I

in der

A der Rest einer Diazo- oder Kupplungskomponente und

R ein Rest der Formel

CN, $COOR^1$, $COSR^1$, $CONH_2$, $CONHNH_2$, $CONH-NH-COR^1$,

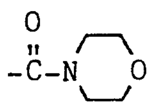 , 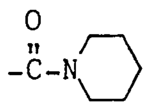 , 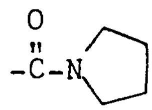 ,

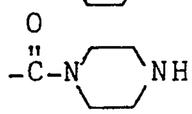 , 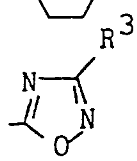 , 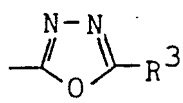 oder 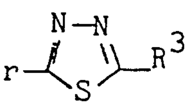

Z Amino,

R und Z zusammen ein Rest der Formel -NHCO-O-CO-,

X Wasserstoff, Chlor, Brom, Methoxy, Äthoxy, Propoxy, $SO_3H$, Butoxy, Phenoxy, Methyl, Äthyl, Propyl, Butyl, Acetylamino, Dimethylamino, Diäthylamino, Carboxyl oder $-NO_2$ sind, wobei

$R^1$ gegebenenfalls substituiertes Alkyl, Alkenyl, Cycloalkyl oder Aryl,

$R^2$ Wasserstoff oder gegebenenfalls substituiertes Alkyl und

$R^3$ Wasserstoff oder einen Rest $R^1$ bedeuten und wobei Aminobenzoesäureamide, Mono- und Dichloraniline als Diazokomponenten A ausgeschlossen sind, wenn $R^1$ $C_1$- bis $C_4$-Alkyl, Methoxyäthyl oder Butoxyäthoxaäthyl ist.

Bg/BL

Die Reste A der Diazokomponenten stammen vorwiegend aus der Anilin-, Aminonaphthalin-, Aminoanthrachinon-, Thiazol-, Benzthiazol-, Benzisothiazol-, Thiadiazol-, Indazol-, Pyrazol- oder Azobenzol-Reihe.

Als Substituenten für die Reste A der Diazokomponente sind beispielsweise zu nennen:

In der Benzolreihe: Chlor, Brom, Nitro, Cyan, Trifluormethyl, Methylsulfonyl, Äthylsulfonyl, Phenylsulfonyl, Carboxyl, Carbomethoxy, Carbobutoxy, Carbo-ß-methoxy-äthoxy, Carbo-ß-hydroxy-äthoxy, gegebenenfalls N-mono- oder N-disubstituiertes Carbon- oder Sulfonamid, Methyl, Äthyl, Methoxy und Äthoxy.

N-Substituenten der Carbon- oder Sulfonamide sind dabei z. B. Methyl, Äthyl, Propyl, Butyl, ß-Hydroxyäthyl, γ-Hydroxy-propyl, ß-Methoxy-äthyl, γ-Methoxy-propyl oder γ-Äthoxypropyl sowie das Pyrrolidid, Piperidid oder Morpholid.

In der Azobenzolreihe: Chlor, Brom, Nitro, Cyan, Methyl, Hydroxy, Äthyl, Methoxy oder Äthoxy. In der heterocyclischen Reihe: Chlor, Brom, Nitro, Cyan, Methyl, Äthyl, Phenyl, Methoxy, Äthoxy, Methylmercapto, ß-Carbomethoxy-äthylmercapto, ß-Carboäthoxy-äthylmercapto, Carbomethoxy, Carboäthoxy, Acetyl, Methylsulfonyl oder Äthylsulfonyl.

Der Rest A leitet sich im einzelnen z. B. von folgenden Aminen ab: o-, m- oder p-Nitroanilin, o-, m- oder p-Cyananilin, 2,4-Dicyananilin, o-, m- oder p-Bromanilin, 2,4,6-Tribromanilin, 2-Chlor-4-nitroanilin, 2-Brom-4-nitroanilin, 2-Cyan-4-nitroanilin, 2-Methylsulfonyl-4-nitroanilin, 2-Methyl-4-nitroanilin, 2-Methoxy-4-nitroanilin, Anilin, 2-, 3- oder 4-Toluidin, 2-, 3- oder 4-

Chloranilin, 2,3-, 2,4-, 2,5-, 3,4- oder 3,5-Dichloranilin, 2-Methyl-4-chloranilin, 2-Methyl-5-chloranilin, 2-Methyl-3-chloranilin, 2-, 3- oder 4-Trifluormethylanilin, 2-Trifluormethyl-4-chloranilin, 2-Methyl-4,5-dichloranilin, 3-Methyl-4-chloranilin, 3-Methyl-6-chloranilin, 3-Methyl-4,6-dichloranilin, 4-Methyl-3-chloranilin, 2-, 3- oder 4-Äthylanilin, 2,3-, 2,4-, 2,5-, 3,4- oder 3,5-Dimethylanilin, 3- oder 4-Acetylaminoanilin, 4-Benzoylaminoanilin, 2,5-Dichlor-4-acetaminoanilin, 3-Acetamino-4-methylanilin, 5-Acetamino-2-methylanilin, 4-Acetamino-2-methylanilin, 5-Chlor-4-acetylamino-2-methylanilin, 5-Chlor-2-methoxyanilin, 4,5-Dichlor-2-methoxyanilin, 3-Chlor-4-methoxyanilin, 1-Aminobenzol-4-methylsulfon, 4-Aminodiphenylsulfon, 1-Amino-2-chlor-4-methylsulfon, 1-Amino-2-chlor-6-brom-4-methylsulfon, 4-Amino-acetophenon, 3,4-Dicyananilin, 4-Aminodiphenyläther, 4-Chlor-2-nitroanilin, 4-Methyl-2-nitroanilin, 4-Methoxy-2-nitroanilin, 2-Chlor-5-aminobenzonitril, 2-Amino-5-chlorbenzonitril, 1-Amino-2-nitrobenzol-4-sulfonsäure-n-butylamid oder -ß-methoxyäthylamid, 2,4-Dinitroanilin, 2,4-Dinitro-6-chloranilin, 2,4-Dinitro-6-bromanilin, 2,4-Dinitro-6-cyananilin, 1-Amino-2,4-dinitrobenzol-6-methylsulfon, 2,6-Dichlor-4-nitroanilin, 2,6-Dibrom-4-nitroanilin, 2-Chlor-6-brom-4-nitroanilin, 2,6-Dicyan-4-nitroanilin, 2-Cyan-4-nitro-6-chloranilin, 2-Cyan-4-nitro-6-bromanilin, 1-Amino-2,6-dibrom-benzol-4-methylsulfon, 1-Amino-2,6-dichlorbenzol-4-methylsulfon, 1-Amino-2,4-dinitrobenzol-6-carbonsäure-methylester oder -ß-methoxyäthylester, 3,5-Dichloranthranilsäurepropylester, 3,5-Dibrom-anthranilsäure-ß-methoxyäthylester, N-Acetyl-p-phenylendiamin, 2-, 3- oder 4-Aminobenzoesäure-methylester, -äthylester, -propylester, -butylester, -isobutylester, -ß-methoxyäthylester, -ß-äthoxy-äthylester, -methyl-diglykolester, -äthyldiglykolester, -methyl-triglykolester, -äthyltriglykol-

ester, -ß-hydroxyäthylester, -ß-acetoxy-äthylester, -ß-(ß'-hydroxy-äthoxy)-äthylester, -ß-hydroxy-propylester, -γ-hydroxy-propylester, -ω-hydroxy-butylester, -ω-hydroxy-hexylester, 5-Nitro-anthranilsäuremethylester, isobutylester, -methyl-diglykolester, -ß-methoxyäthylester, -ß-butoxy-äthylester, -ß-acetoxy-äthylester, 3- oder 4-Aminophthalsäure-, 5-Aminoisophthalsäure- oder Amino-terephthalsäure-dimethylester, -diäthylester, -dipropylester, -dibutylester, 3- oder 4-Aminobenzoesäureamid, -methylamid, -propylamid, -butylamid, -isobutylamid, -cyclohexylamid, -ß-äthyl-hexylamid, -γ-methoxypropylamid, -γ-äthoxy-propylamid, 2-, 3- oder 4-Aminobenzoesäure-dimethylamid, -diäthylamid, -pyrrolidid, -morpholid, N-methyl-N-ß-hydroxy-äthylamid, 5-Amino-isophthalsäurediamid, -bis-γ-methoxy-propylamid, Aminoterephthalsäure-bis-diäthylamid, 3- oder 4-Amino-phthalsäure-imid, -ß-hydroxy-äthylamid-, γ-hydroxy-propylamid, 3-Amino-6-nitro-phthalsäure-ß-hydroxy-äthylamid, 2-, 3- oder 4-Aminobenzosulfonsäure-dimethylamid, -diäthylamid, -pyrrolidid, -morpholid, Methylsulfonsäure-2'-, -3'- oder 4'-amino-phenylester, Äthylsulfonsäure-2'-, -3'- oder -4'-amino-phenylester, Butylsulfonsäure-2'-, -3'- oder -4'-aminophenylester, Benzolsulfonsäure-2'-, -3'- oder 4'-aminophenylester, 1- und 2-Amino-anthrachinon, 1-Amino-4-chlor-anthrachinon, 3- oder 4-Aminodiphenylenoxid, 2-Amino-benzthiazol, 2-Amino-6-carbonsäure-methylester-benzthiazol, 2-Amino-6-methyl-sulfonyl-benzthiazol, 2-Amino-6-cyanbenzthiazol, 2-Amino-6-nitro-benzthiazol, 5,6- oder 6,7-Dichlor-2-amino-benzthiazol, 4-Amino-5-brom-7-nitro-1,2-benzisothiazol, 3-Amino-5-nitro-2,1-benzisothiazol, 3-Amino-5-nitro-7-brom-2,1-benzisothiazol, 2-Aminothiazol, 2-Amino-5-nitrothiazol, 2-Amino-4-methyl-thiazol-5-carbonsäure-äthylester, 2-Amino-4-methyl-5-acetyl-thiazol, 2-Amino-3-cyan-4-methyl-thiophen-5-carbonsäureester, 2-Phenyl-5-amino-1,3,4-thiadiazol, 3-Methylmercapto-5-amino-1,2,4-thiadiazol, 3-ß-Carbomethoxy-äthylmercapto-5-amino-1,2,4-thiadiazol sowie von den Diazokomponenten der Formeln

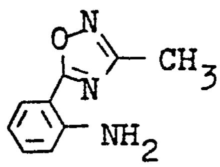 , 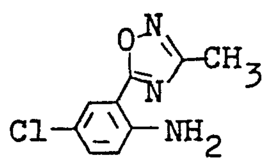 , 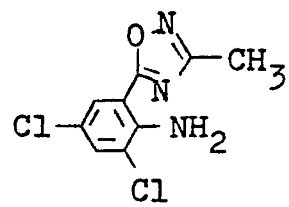 ,

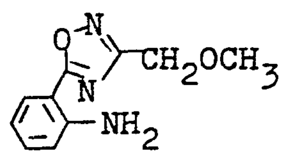 , 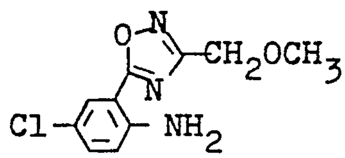 ,

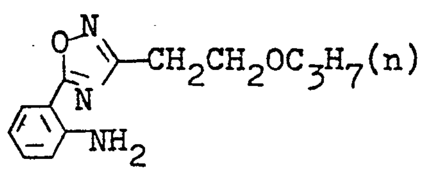 , 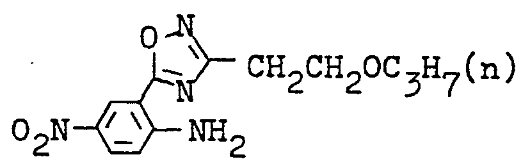

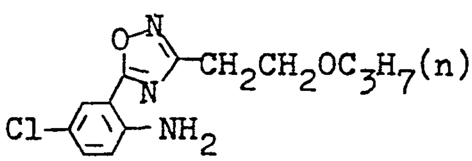 , 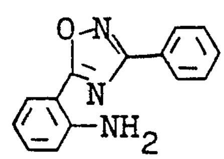 ,

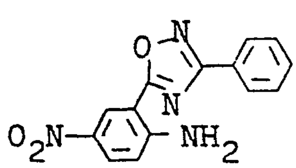 , 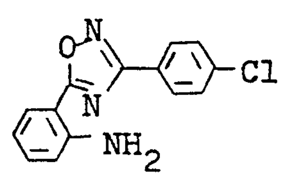 , 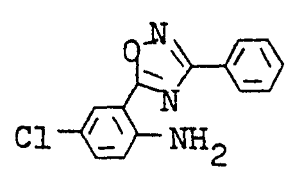 ,

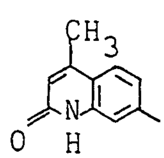 , 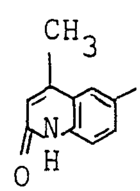 , 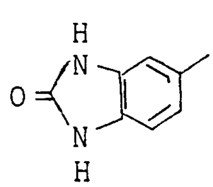 , 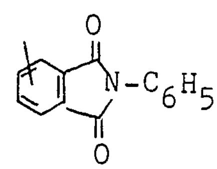 ,

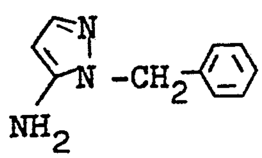 , 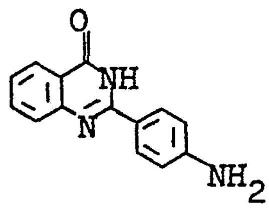 , 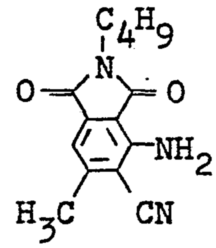

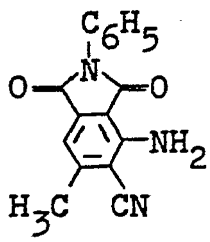 , 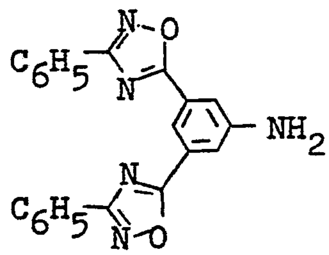 , 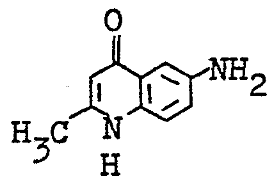 ,

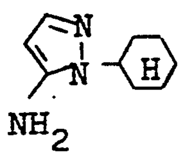 oder 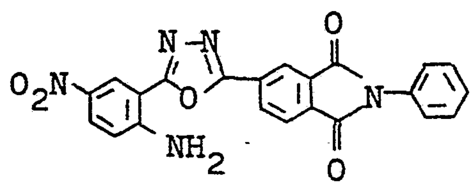 .

Geeignete Diazokomponenten der Aminoazobenzolreihe A-$NH_2$ sind beispielsweise: 4-Aminoazobenzol, 2',3-Dimethyl-4-aminoazobenzol, 3',2-Dimethyl-4-aminoazobenzol, 2,5-Dimethyl-4-aminoazobenzol, 2-Methyl-5-methoxy-4-aminoazobenzol, 2-Methyl-4',5-dimethoxy-4-aminoazobenzol, 4'-Chlor-2-methyl-5-methoxy-4-aminoazobenzol, 4'-Nitro-2-methyl-5-methoxy-4-aminoazobenzol, 4'-Chlor-2-methyl-4-aminoazobenzol, 2,5-Dimethoxy-4-aminoazobenzol, 4'-Chlor-2,5-dimethoxy-4-aminoazobenzol, 4'-Nitro-2,5-dimethoxy-4-aminoazobenzol, 4'-Chlor-2,5-dimethyl-4-aminoazobenzol, 4'-Methoxy-2,5-dimethyl-4-aminoazobenzol, 4'-Nitro-4-aminoazobenzol, 3,5-Dibrom-4-aminoazobenzol, 2,3'-Dichlor-4-aminoazobenzol, 3-Methoxy-4-aminoazobenzol, 4'-Hydroxy-2'-methyl-4-aminoazobenzol, 3-Chlor-4-aminoazobenzol.

Von den besonders wertvollen Diazokomponenten A-$NH_2$ seien im einzelnen genannt: 4-Nitro-anilin, 2-Chlor-4-nitroanilin, 2-Brom-4-nitroanilin, 2-Cyan-4-nitro-anilin, 2-Methoxy-4-nitro-anilin, 2-Amino-5-nitro-phenylsulfonsäuredimethylamid, 2-Amino-5-nitro-phenylsulfonsäurebutylamid, 2-Amino-5-nitro-phenylsulfonsäure-ß-methoxyäthylamid, 2-Amino-benzonitril, 2-Chlor-4-amino-benzonitril, 2-Chlor-5-amino-benzonitril, 2-Amino-5-chlor-benzonitril, 3,5-Dichlor-2-amino-benzonitril, 1-Amino-2,4-dicyanbenzol, 1-Amino-2,4-dicyan-6-chlor-benzol, 2-Chlor-4-amino-5-nitro-benzonitril, 2-Amino-3-chlor-5-nitrobenzonitril, 2-Amino-3-brom-5-nitro-benzonitril, 2,6-Dicyan-4-nitro-anilin, 2,5-Dichlor-4-nitroanilin, 2,6-Dichlor-4-nitro-anilin, 2,6-Dibrom-4-nitro-anilin, 2-Chlor-6-brom-4-nitro-anilin, 2,4-Dinitro-anilin, 2,4-Dinitro-6-chloranilin, 2,4-Dinitro-6-brom-anilin, 2-Amino-3,5-dinitro-benzonitril, 1-Amino-4-nitrobenzol-2-methylsulfon, 1-Amino-4-nitrobenzol-2-äthylsulfon, 4-Methylsulfonyl-anilin, 1-Amino-2-chlorbenzol-4-methylsulfon, 1-Amino-2,6-dibrombenzol-4-methylsulfon, 1-Amino-2,6-dichlorbenzol-4-methylsulfon, 2-(3'-Phenyl-oxdiazolyl-1,2,4)-anilin, 2-(3'-Methoxymethyloxdiazolyl-1,2,4)-anilin, o-, m-, p-Chloranilin, o-, m-, p-Bromanilin, Anthranilsäure-methyl-, äthyl-, propyl-, methoxyäthylester, 2- und 4-Amino-benzoesäureester, 2-Amino-5-nitrobenzoesäureester, 2-Amino-3-chlor-5-nitro-benzoesäureester, 2-Amino-3,5-dichlor-benzoesäureester, 2-Amino-3,5-dibrom-benzoesäureester, 2-Amino-3,5-dinitro-benzoesäure-methylester oder -ß-methoxy-äthylester, 2-Amino-

terephthalsäure-diäthylester, 4-Amino-azobenzol, 2,3'-Dimethyl-4-amino-azobenzol, 2',3-Dimethyl-4-amino-azobenzol, 2,5-Dimethyl-4-amino-azobenzol, 3,5-Dibrom-4-amino-azobenzol, 3-Chlor-4-aminoazobenzol, 3-Brom-4-aminoazobenzol, p-Cyananilin, 2,6-Dicyan-4-chlor- oder -4- methylanilin oder 2,6-Dichlor-4-cyananilin.

Von den besonders wertvollen heterocyclischen Diazokomponenten A-$NH_2$ seien erwähnt: 2-Amino-thiazol, 2-Amino-5-nitro-thiazol, 2-Amino-4-methyl-thiazol-5-carbonsäure-äthylester, 2-Amino-5-phenyl-1,3,4-thiadiazol, 3-Phenyl-5-amino-1,2,4-thiadiazol, 3-Methyl-mercapto-5-amino-1,2,4-thiadiazol, 3-ß-Carbomethoxy-äthylmercapto-5-amino-1,2,4-thiadiazol, 3-ß-Carboäthoxy-äthylmercapto-5-amino-1,2,4-thiadiazol, 2-Amino-6-cyan-benzthiazol, 2-Amino-6-carbonsäuremethylester-benzthiazol, 2-Amino-6-nitro-benzthiazol, 2-Amino-3-cyan-4-methyl-thiophen-5-carbonester, 1-Benzyl-5-aminopyrazol, 1-Cyclohexyl-5-aminopyrazol.

Kupplungskomponentenreste A stammen z. B. aus der Phenol-, Naphthalin-, Pyridon-, Pyrazolon-, Pyridin-, Pyrimidin-, Acetoacetanilid-, Indol-, Thiazol-, Homophthalimid- oder Anilinreihe.

Einzelne repräsentative Beispiele sind z. B.: Phenol, o-, m-, p-Kresol, 2,6-Dihydroxy-4-methylpyridin, 2,6-Dihydroxy-3-cyan-4-methyl-pyridin, 1,4-Dimethyl-2-hydroxy-3-cyan-pyridon-6, 1-Äthyl-2-hydroxy-3-cyan-4-methyl-pyridon-6, 2-Amino-naphthalin-1-sulfosäure, 1-Hydroxynaphthalin, 2-Hydroxynaphthalin, 2-Naphthol-3-carbonsäurephenylamid, 2-Naphthol-3-carbonsäure-anisidid, 3-Phenyl-5-diäthylamino-thiazol, Acetessigsäuredianisidid, Phenylindol, Methylindol, 1-Phenyl-3-methylpyrazolon-5 oder 1-Phenyl-3-carbo-$C_1$- bis $C_4$-alkoxy-pyrazolon-5.

Einzelne Reste R der Formel $COOR^1$ sind z.B.:

$COOCH_3$, $COOC_2H_5$, $COOCH(CH_3)_2$, $COOC_3H_7(n)$, $-COOCH(C_2H_5)-CH_3$, $COOC_4H_9(n)$, $COOC_5H_{11}$, $COOC_6H_{13}(n)$, $COOC_6H_{13}(i)$, $COOCH_2-CH(C_2H_5)-C_4H_9(n)$, $COOC_9H_{19}(n)$, $COOC_{10}H_{21}(n)$, $COOC_{10}H_{21}(i)$, $COOCH_2CH_2OCH_3$, $COOCH_2CH_2OC_2H_5$, $COOCH_2CH_2OC_4H_9(n)$, $COOCH_2CH_2OC(CH_3)(C_2H_5)$, $CO(OCH_2CH_2)_2OCH_3$, $CO(OCH_2CH_2)_2OC_4H_9(n)$, $CO(OCH_2CH_2)_3OCH_3$, $CO(OCH_2CH_2)_3OC_4H_9(n)$, $CO(OCH_2CH_2)_4OCH_3$, $CO(OCH_2CH_2)_4OC_4H_9(i)$, $CO(OCH_2CH(CH_3))_2OCH_3$, $CO(OCH(CH_3)CH_2)_2OCH_3$,

$COOCH(CH_3)CH=CH_2$, $COOCH_2CH_2CH(OCH_3)CH_3$, 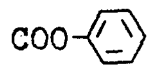 , 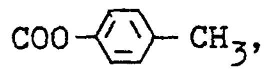 ,

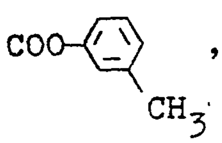 , 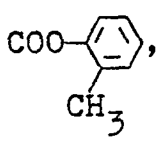 , 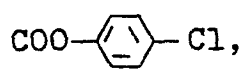 , 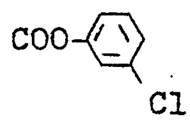 , 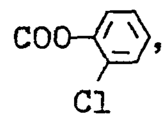 ,

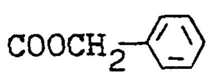 , 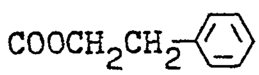 , 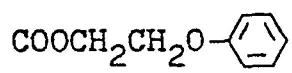 ,

$COOCH_2CH_2OCOCH_3$, $COOCH_2CH_2NHCOCH_3$, $COO(CH_2)_3NH-COCH_3$,

Reste $R^3$ an den heterocyclischen Resten

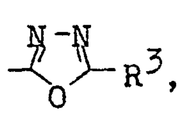 , 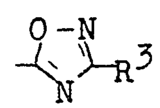 oder 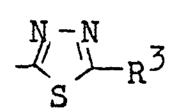

sind beispielsweise: Wasserstoff, gegebenenfalls durch Sauerstoff unterbrochenes und durch Hydroxy, Pyrrolidonyl, Phthalimidyl, $C_1$- bis $C_8$-Alkoxy, Allyloxy, Benzyloxy, Phenyläthoxy, Phenoxy, Methylphenoxy, Chlorphenoxy oder Methoxyphenoxy substituiertes $C_1$- bis $C_8$-Alkyl, Benzyl, Phenyläthyl, Cyclohexyl, gegebenenfalls durch Chlor,

Brom, Nitro, Methyl, Äthyl, Methoxy oder Äthoxy substituiertes Phenyl, Naphthyl, Pyridyl, Thienyl oder Furyl.

Einzelne Reste $R^3$ der heterocyclischen Reste sind beispielsweise: Methyl, Äthyl, Propyl, Butyl, Pentyl, Hexyl, Heptyl, 1-Äthylpentyl, Methoxymethyl, Methoxyäthyl, (n)-Propoxyäthyl, (i)-Propoxyäthyl, (n)-Butoxyäthyl, (n)-Hexoxyäthyl, Phenyl, Phenylallyl oder Reste der Formeln

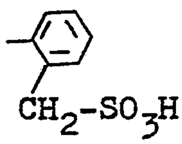 , $-CH_2CH_2-SO_3H$, $-CH_2-SO_3H$, 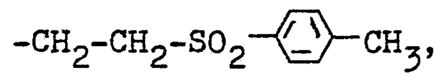,

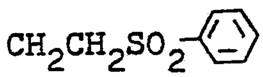 .

Zur Herstellung der Verbindungen der Formel I, in denen A der Rest einer Diazokomponente ist, kann man eine Diazoverbindung von Aminen der Formel

$$A-NH_2$$

mit einer Verbindung der Formel

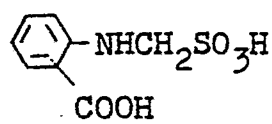

kuppeln, anschließend den Rest $CH_2SO_3H$ wieder abspalten und die COOH-Gruppe in einen Rest der Formel R überführen.

Zur Herstellung der Verbindungen der Formel I, bei denen A der Rest einer Kupplungskomponente ist, kann man Verbindungen der Formel

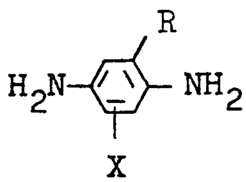

mit einer Verbindung der Formel

AH

kuppeln.

Die Verfahren sind im Prinzip bekannt. Einzelheiten der Herstellung können den Beispielen entnommen werden, in denen sich Angaben über Teile und Prozente, sofern nicht anders vermerkt, auf das Gewicht beziehen.

Die Verbindungen der Formel I sind wertvolle Diazokomponenten zur Herstellung von Azofarbstoffen, die man daraus durch Diazotierung und Kupplung mit Kupplungskomponenten erhält.

Von besonderer Bedeutung sind Verbindungen der Formel I a

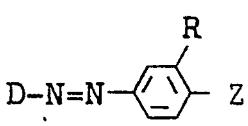

in der

D der Rest einer Diazokomponente ist und Z und

R die angegebene Bedeutung haben.

Bevorzugte Diazokomponenten entstammen der Anilin-, Phthalimidyl-, Chinazolonyl-, Pyrazol- und Thiazol-Reihe, wobei als Substituenten für die Diazokomponenten insbesondere Fluor, Chlor, Brom, Trifluormethyl, Methyl,

Äthyl, Methoxy, Äthoxy, Phenoxy, Cyan, Nitro, Methylsulfonyl, Äthylsulfonyl, Allylsulfonyl, Phenylsulfonyl, Carboxyl, Carbonsäureester, gegebenenfalls substituiertes Sulfamoyl oder Oxdiazolyl in Betracht kommen.

Bevorzugte Reste R sind

$COOR^1$, 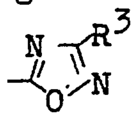 und 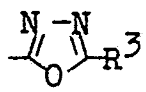 .

Bevorzugte Reste $R^1$ sind beispielsweise: $COOCH_3$, $COOC_2H_5$, $COOC_3H_7(n)$, $COOC_3H_7(i)$, $COOC_4H_9(n)$, $COO-CH(C_2H_5)(CH_3)$, $COOC_5H_{11}$, $COOC_6H_{13}(n)$, $COOC_6H_{13}(i)$, $COOC_8H_{17}(n)$, $COOCH_2CH(C_2H_5)-C_4H_9(n)$, $COOC_9H_{19}(n)$, $COOC_{10}H_{21}(n)$, $COOC_{10}H_{21}(i)$, $COOCH_2CH_2OCH_3$, $COOCH_2CH_2OC_4H_9$, $CO(OCH_2CH_2)_2OCH_3$, $CO(OCH_2CH_2)_2OC_2H_5$, $CO(OCH_2CH_2)_2OC_4H_9(n)$, $CO(OCH_2CH_2)_3OCH_3$, $CO(OCH_2CH_2)_3OC_4H_9$, $CO(OCH_2CH_2)_4OCH_3$, $CO(OCH_2CH_2)_4OC_4H_9$, COO-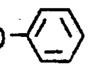 , $COOCH_2$-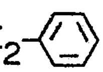 , $CON(CH_3)_2$, $CON(C_2H_5)_2$, $CONH_2$.

Bevorzugte Reste $R^3$ in der 1,2,4-Oxdiazolyl-Reihe sind beispielsweise: $CH_3$, $C_2H_5$, $CH_2OCH_3$, $CH_2CH_2OCH_3$, $CH_2CH_2OC_2H_5$, $CH_2CH_2OC_3H_7(n)$, $(CH_2CH_2O)_2CH_3$, $(CH_2CH_2O)_3CH_3$, $(CH_2CH_2O)_2C_3H_7(n)$, $C_6H_5$, $C_6H_4CH_3$ oder $C_6H_4Cl$.

Bevorzugte Reste $R^3$ in der 1,3,4-Oxdiazolyl-Reihe sind beispielsweise: $CH_3$, $C_2H_5$, $C_3H_7$, $C_5H_{11}(n)$, $CH(C_2H_5)C_4H_9(n)$, $C_6H_{13}$ oder $C_6H_5$.

Beispiel 1

127,5 Teile 3-Chlor-anilin werden auf übliche Art und Weise wäßrig diazotiert. Nach Zerstören der überschüssigen salpetrigen Säure mit Amidosulfonsäure puffert man die Diazoniumsalzlösung z. B. mit Hilfe von Natriumhydrogencarbonat auf pH 5,0 bis 6,0 ab und setzt dann eine Lösung von 232 Teilen der Verbindung der Formel

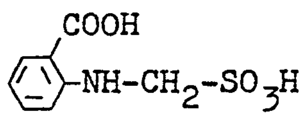

in 750 Raumteilen Wasser zu. Die Kupplung rührt über Nacht bei pH 4,5 bis 6,0 und ist dann beendet. Nun hebt man den pH-Wert des Gemisches durch Zugabe von 80 Teilen einer 50 %igen Natronlauge auf pH 10,0 bis 11,0 an und erhitzt auf 80 °C, wobei man insgesamt noch 80 Teile einer 50 %igen Natronlauge zusetzt, damit der pH-Wert nicht unter 9,8 abfällt. Nach etwa 3 Stunden ist die Abspaltung der Methansulfonsäuregruppe beendet. Man filtriert das ausgefallene Produkt ab. Das Filtergut wird nochmals mit 1000 Raumteilen Wasser angerührt, mit Salzsäure auf pH 5,0 bis 7,5 angesäuert und dann erneut abgesaugt. Nach dem Waschen mit Wasser und Trocknen erhält man 240 Teile eines gelbbraunen Pulvers der Formel

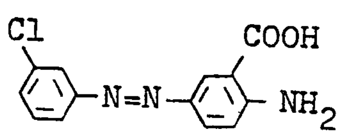

das sich in Aceton rückstandsfrei löst und bei 230 - 235 °C schmilzt.

UV-Spektrum: $\lambda_{max.}$: 371 nm

in Eisessig $\varepsilon_{max.}$: 191513 $\frac{L}{Mol \cdot cm}$

455 Teile 4-Amino-3'-chlor-azobenzol-3-carbonsäure werden in 2000 Raumteilen Nitrobenzol suspendiert. Bei 90/95 °C leitet man 5 Stunden lang Phosgen ein, anschließend wird überschüssiges Phosgen durch Einblasen von Luft in das Reaktionsgemisch entfernt und das ausgefallene Produkt der Formel

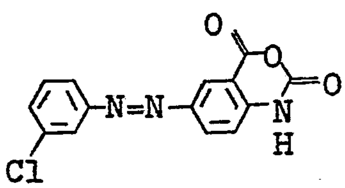

abgesaugt, mit Cyclohexan gewaschen und getrocknet. Man erhält 360 Teile eines braunen Pulvers, dessen Schmelzpunkt ⟩ 150 °C ist.

Cl-Bestimmung: gef. 12,5 %, Theorie 11,8 %

150 Teile der so erhaltenen Verbindung werden mit 350 Raumteilen N-Methyl-pyrrolidon und 72 Teilen 2-Äthylhexanol auf 80 °C erwärmt. Dann tropft man 15 Raumteile Triäthylamin zu und rührt das Gemisch 4 Stunden bei 110 °C. Nach dem Erkalten läßt man unter guter Rührung langsam 1000 Raumteile Wasser hinzulaufen und filtriert das ausgefallene Produkt der Formel

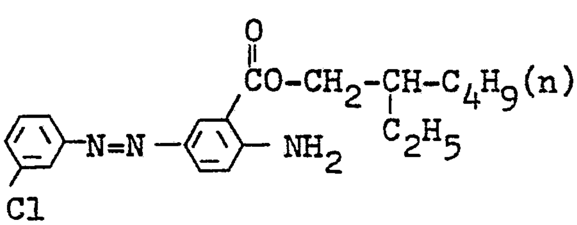

ab, wäscht es mit Wasser und trocknet. Man erhält 180 Teile eines goldgelben Pulvers, das bei 93 - 96 °C

schmilzt.

UV-Spektrum: $\lambda_{max.}$: 370 nm

$\varepsilon_{max.}$: 230620 $\frac{L}{Mol \cdot cm}$

Schulter bei 349 nm mit $\varepsilon$: 213180 $\frac{L}{Mol \cdot cm}$

Lösungsmittel: Eisessig.

Beispiel 2

500 Teile der Verbindung der Formel

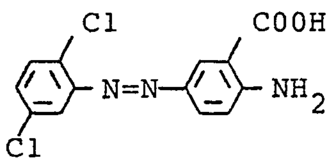

(Absorptionsmaximum in Dimethylformamid 416 nm)

(die analog der in Beispiel 1 beschriebenen Verfahrensweise hergestellt wurden) werden analog der in Beispiel 1 beschriebenen Arbeitsweise phosgeniert, man erhält 510 Teile der Verbindung der Formel

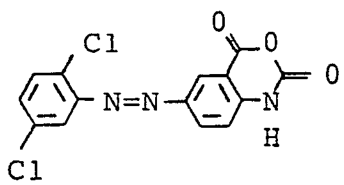

mit einem Schmelzpunkt von 327 °C.

100 Teile dieses Produktes werden mit 200 Raumteilen Aceton versetzt und mit 1000 Teilen Ammoniakwasser (25 %ig) vereinigt. Man rührt das Gemisch 1 Stunde bei 50 °C, filtriert dann ab und wäscht mit Wasser. Nach dem Trocknen erhält man 78 Teile des Produktes der Formel

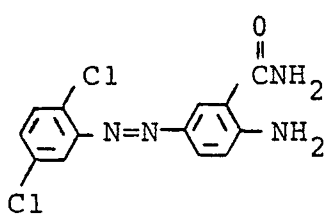

mit einem Schmelzpunkt von 232 °C.
IR-Spektrum: sehr starke Absorptionsbanden bei 1595 und 1623 $cm^{-1}$.

72 Teile dieses Produktes werden in 240 Teilen Dimethylformamid gelöst und das Gemisch zu einer Lösung von 60 Teilen Phosgen in 160 Teilen Dimethylformamid gegeben. Man rührt das Gemisch bei 40 °C 1 Stunde nach und läßt es dann nach Erkalten auf 300 Teile Wasser ausrühren. Die erhaltene Suspension wird mit Natronlauge auf einen pH-Wert von 7 - 8 abgepuffert. Nach Absaugen und Waschen des ausgefallenen Produktes isoliert man 75 Teile eines gelben Pulvers der Formel

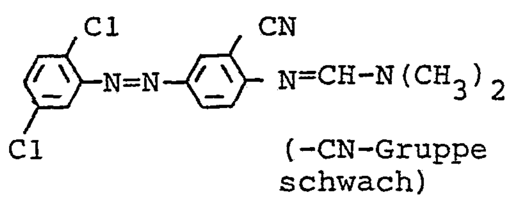

(-CN-Gruppe im IR bei 2200 $cm^{-1}$ schwach)

Das Produkt hat keinen scharfen Schmelzpunkt und sintert zwischen 160 bis 250 °C. 70 Teile dieses Produktes werden mit 400 Raumteilen Methanol und 80 Teilen konzentrierter Schwefelsäure sowie mit 50 Teilen Wasser versetzt. Man rührt das Gemisch 8 Stunden unter Siedekühlung, anschließend werden 3000 Teile Wasser zugesetzt und die Fällung mit Natronlauge auf pH 3,5 - 4,5 abgestumpft. Das ausgefallene Produkt wird heiß abfiltriert und mit Wasser gewaschen. Man erhält 60 Teile 2',5'-Dichlor-3-cyan-4-aminoazobenzol mit einem Schmelzpunkt von 250 °C. Die Cyangruppe erscheint in der spektralphotometrischen IR-Aufnahme bei 2195 $cm^{-1}$ (mittel ausgeprägt).

Beispiel 3

Setzt man analog der in Beispiel 2 beschriebenen Arbeitsweise 100 Teile der Verbindung der Formel

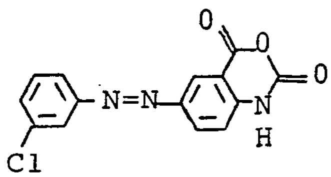

mit Ammoniak um, so erhält man 74 Teile 3'-Chlor-3-carbamoyl-4-aminoazobenzol (Schmelzpunkt: 252 °C, IR-Spektrum: sehr starke, gespaltene Absorptionsbande bei 1591 $cm^{-1}$ und 1619 $cm^{-1}$).

Durch Entwässerung (z. B. analog dem im Beispiel 2 beschriebenen Verfahren) erhält man daraus 66 Teile 3'-Chlor-3-cyan-4-amino-azobenzol mit einem Schmelzpunkt von 255 °C (IR-Spektrum: -CN-Bande bei 2190 $cm^{-1}$ (schwach), weitere starke Absorptionsbanden bei z. B. 1685 $cm^{-1}$, 1589 $cm^{-1}$ (Träger: KBr)).

Beispiel 4

353 Teile 4-Aminobenzoesäure-äthylester werden wie üblich in ca. 2000 Raumteilen Wasser mit Salzsäure und Natriumnitrit unter Zusatz von Eis diazotiert. Dann läßt die Diazoniumsalzlösung nach Entfernen von überschüssiger salpetriger Säure zu einer Lösung von 445 Teilen der Verbindung der Formel

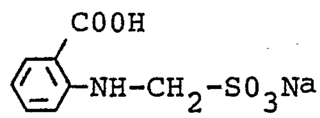

in 1500 Raumteilen Wasser von 0 bis 10 °C ab. Der pH-Wert der Lösung der Kupplungskomponente wurde zuvor auf 4,5 bis 6,0 abgepuffert. Während des Zulaufs der Diazoniumsalzlösung hält man das Kupplungsgemisch bei pH 4,5 bis 6,0 (z. B. indem man festes Natriumbicarbonat, Natriumcarbonat oder verdünnte Natronlauge zusetzt). Die Kupplung rührt 10 Stunden nach und erwärmt sich dabei auf Raumtemperatur. Dann heizt man das Gemisch auf 70 - 90 °C auf und setzt analog der im Beispiel 1 beschriebenen Arbeitsweise konzentrierte Natronlauge zu, so daß die Schutzgruppe bei einem pH-Wert von 10 bis ca. 13 innerhalb von 3 bis 4 Stunden abgespalten wird. Anschließend läßt man erkalten, säuert mit Salzsäure auf pH 4 bis 5 an, filtriert ab, wäscht und trocknet.

Man erhält 495 Teile eines dunkelgelben Produktes der Formel

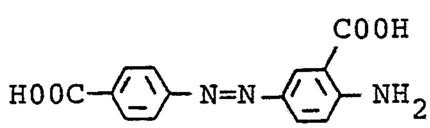

das sich beim Erwärmen bei 260 °C zersetzt. Eine gelbe Lösung von 0,01 Teilen dieses Produktes in 1000 Raumteilen Dimethylformamid hat ein Absorptionsmaximum bei 407,5 Nanometer (nm).

373 Teile dieses Produktes werden in 1000 Raumteilen Nitrobenzol und 15 Teilen Tetrahydrofuran suspendiert. Anschließend gast man bei 40 bis 70 °C solange Phosgen ein, bis die Umsetzung beendet ist (Dauer 3 - 6 Stunden). Nach Ausblasen überschüssigen Phosgens wird ausgefallenes Produkt abfiltriert und mit Cyclohexan gewaschen. Nach Trocknen erhält man 446 Teile der Verbindung der Formel

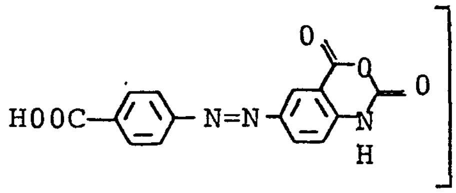 · HCl

als braunes Pulver mit einem Schmelzpunkt von 132 °C.

100 Teile dieser Verbindung werden analog der in Beispiel 1 beschriebenen Arbeitsweise mit 300 Teilen n-Propanol umgesetzt. Nach Isolieren und Trocknen wie üblich erhält man ein gelbes Pulver der Formel

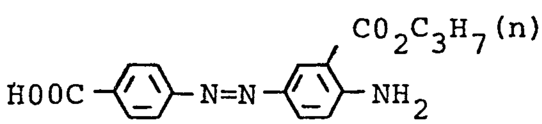

das sich in Dimethylformamid mit schwach gelber Farbe löst und ein Absorptionsmaximum von 401 nm hat.

Beispiel 5

121 Teile des Produktes der Formel

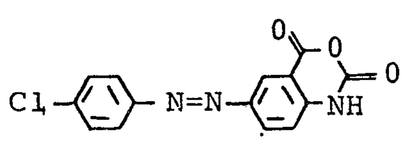

werden in 400 Raumteilen N-Methylpyrrolidon suspendiert. Dann setzt man 60 Teile 2-Phenoxyäthanol hinzu, erhitzt auf 50 - 80 °C und tropft noch 5 Teile Triäthylamin hinzu. Man rührt 2 - 3 Stunden bis zur beendeten Reaktion nach, kühlt auf Raumtemperatur und fällt das Reaktionsprodukt mit Wasser aus.

Nach Absaugen, Waschen mit Wasser und Trocknen erhält man 148 Teile der Diazokomponente der Formel

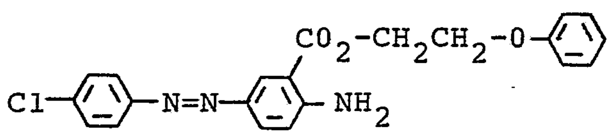

als gelbbraunes Pulver, das bei 140 °C schmilzt.

Beispiel 6

150 Teile der Verbindung der Formel

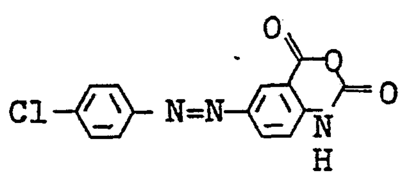

werden mit 800 Teilen Methanol und 20 Raumteilen Triäthylamin analog zu der in Beispiel 1 beschriebenen Weise behandelt. Man erhält 141 Teile der Verbindung der Formel

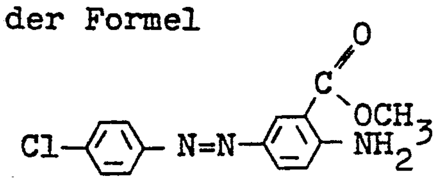

max. (DMF): 413 nm

als gelbes Pulver, das bei 208 - 210 °C schmilzt.

140 Teile der gleichen Verbindung erhält man, wenn anstatt Triäthylamin 27 Teile Natriummethylat in 80 Raumteilen Methanol zugesetzt werden und ansonsten analog verfahren wird.

Beispiel 7

353 Teile 4-Aminobenzoesäureäthylester werden analog zu der in Beispiel 5 beschriebenen Arbeitsweise zu 4'-Carboxyäthyl-3-carboxyl-azobenzol-4-amino-ω-methansulfonsäure

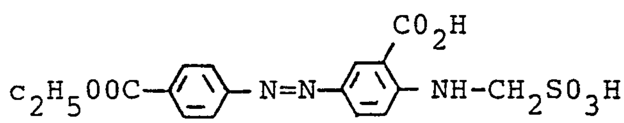

gekuppelt. Führt man nun die Abspaltung der Schutzgruppe bei 70 - 95 °C sauer durch (z. B. in ca. 80 %iger Ameisensäure), so erhält man nach der üblichen Isolierung 544 Teile des Produktes der Formel

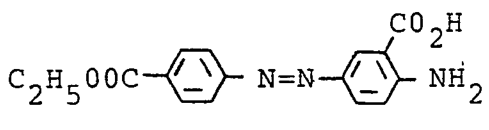

als gelbbraunes Pulver mit einem Schmelzpunkt von 210 °C. Das Absorptionsmaximum in Dimethylformamid bei einer Konzentration von 0,01 Teilen in 1000 Raumteilen Dimethylformamid befindet sich bei 413 nm. 289 Teile dieses Produktes werden in 1400 Raumteilen Chloroform unter Zusatz von 50 Raumteilen Tetrahydrofuran bei 40 - 50 °C wie üblich phosgeniert. Nach Absaugen und Trocknen erhält man 301 Teile der Verbindung der Formel

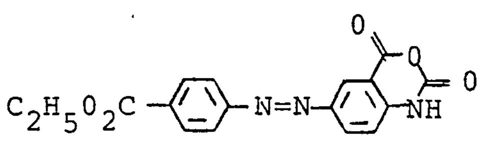

als braunes Pulver (Absorptionsmaximum in Dimethylformamid: 419 nm).

158 Teile dieses Produktes werden in ein Gemisch aus 700 Teilen Äthylalkohol und 10 Teilen Triäthylamin gegeben. Man rührt 1 1/2 Stunden bei 50 bis 60 °C nach, destilliert überschüssigen Alkohol soweit wie möglich ab und isoliert das entstandene Produkt nach Zugabe von Wasser wie üblich. Nach dem Trocknen erhält man 150 Teile der Diazokomponente der Formel

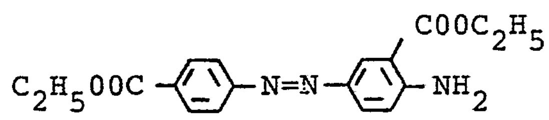

die sich in Dimethylformamid mit schwach gelber Farbe löst und ein Absorptionsmaximum von 406 nm hat.

Beispiel 8

34,6 Teile der Verbindung der Formel

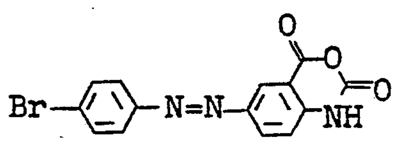

werden in 250 Raumteilen N-Methylpyrrolidon verrührt. Dazu gibt man bei 50 °C 8 Teile Diäthylamin und rührt die erhaltene Lösung 2 Stunden bei 80 °C. Anschließend fällt man das Reaktionsgemisch auf Eiswasser. Das ausgefallene Produkt der Formel

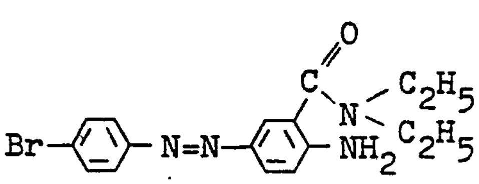

wird wie üblich isoliert. Man erhält ein gelbes Pulver, das bei 135 °C schmilzt.

Beispiel 9

13,6 Teile der Verbindung der Formel

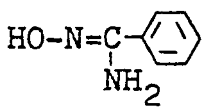

werden in 100 Raumteilen Wasser und 32 Raumteilen Isobutanol gelöst und auf 50 °C erwärmt. Dann gibt man 30,15 Teile der Verbindung der Formel

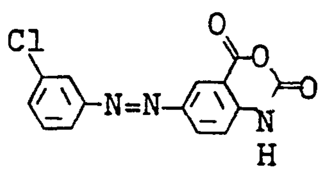

hinzu und rührt bei 50 - 70 °C. Nach beendeter $CO_2$-Abspaltung heizt man auf 80 °C auf und gibt 50 %ige Natronlauge hinzu, so daß der pH-Wert des Gemisches auf 11 - 13 steigt. Man rührt 30 Minuten nach, destilliert Isobutanol ab und isoliert das ausgefallene Produkt durch Absaugen. Nach Waschen mit Wasser und Trocknen erhält man 31 Teile der Verbindung der Formel

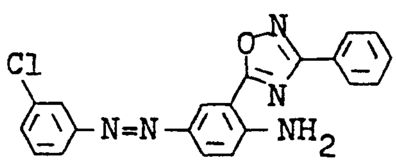

als gelbes Pulver, das bei 172 - 175 °C schmilzt.

Beispiel 10

95 Teile der Verbindung der Formel

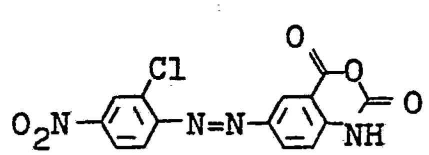

(sandfarbenes Pulver, Schmelz- bzw. Zers.Punkt 260 - 265 °C)

werden analog der in Beispiel 4 beschriebenen Arbeitsweise mit Äthanol und Triäthylamin umgesetzt und aufgearbeitet. Man erhält 90 Teile eines roten Pulvers der Formel

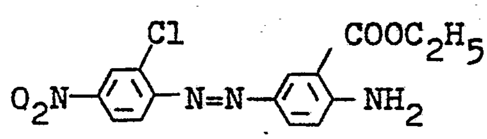

das bei 173 °C schmilzt.

Beispiel 11

20,8 Teile der Verbindung der Formel

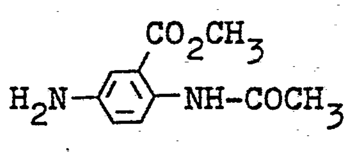

werden mit 200 Raumteilen Eisessig und 30 Raumteilen Salzsäure (30 %ig) versetzt. Man kühlt das Gemisch durch Zugabe von 200 Teilen Eis ab, setzt dann 33 Raumteile einer 23 %igen Natriumnitritlösung zu und diazotiert 3 Stunden bei 0 bis 5 °C. Nach Zerstören von überschüssiger salpetriger Säure tropft man die Diazoniumsalz-Lösung zu einer auf 0 - 10 °C abgekühlten Lösung von 10 Teilen Phenol in 200 Raumteilen Wasser und 50 Teilen Natriumacetat. Nach Rühren über Nacht isoliert man das entstandene Produkt der Formel

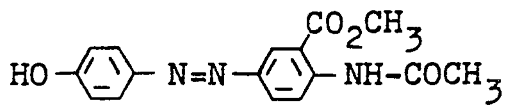

wie üblich und spaltet anschließend durch 4stündiges Kochen des erhaltenen Produktes in 300 Raumteilen Methanol und 10 Raumteilen konzentrierter Salzsäure die Acetylverbindung ab. Die Isolierung der Aminoazoverbindung erfolgt durch Abkühlen des Reaktionsgemisches im Eisbad und Absaugen des ausgefällten Produkts. Nach Waschen und Trocknen erhält man 13 Teile der Verbindung der Formel

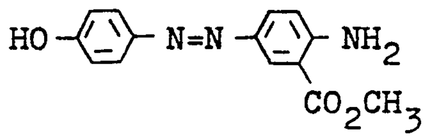

als schwarzes Pulver, das bei 205 - 212 °C schmilzt.

Beispiel 12

59 Teile der Verbindung der Formel

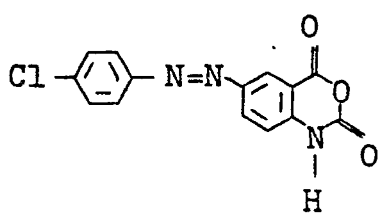

und 16 Teile Essigsäurehydrazid werden in 100 Teilen N-Methylpyrrolidon 3 Stunden auf 70 °C erhitzt. Nach Abkühlen wird mit 300 Teilen Wasser verdünnt, abgesaugt, mit Wasser gewaschen und getrocknet. Man erhält 64 Teile der Verbindung der Formel

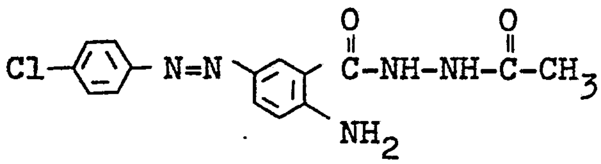

58 Teile dieser Verbindung werden bei 0-°C in 210 ml 23 %iges Oleum unter Zusatz von 4 g Borsäure eingetragen, 1 Stunde bei 5 - 10°C und dann 10 Stunden bei 24°C gerührt. Die Reaktionslösung wird auf Eis gegossen, abgesaugt, mit Wasser gewaschen und getrocknet. Man erhält 53 Teile der Verbindung der Formel

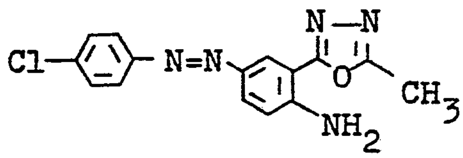

als gelbes kristallines Pulver mit Schmelzpunkt 220 - 223 °C.

Beispiel 13

27 Teile der Verbindung der Formel

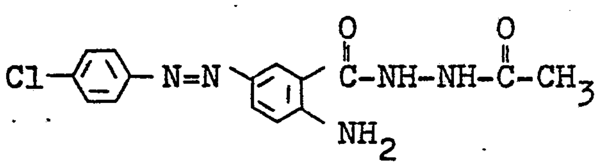

werden in 100 Teilen N-Methylpyrrolidon mit 13 Teilen $P_2O_5$ versetzt und 4 Stunden bei 100 °C gerührt. Man verdünnt danach mit 300 Teilen Wasser, saugt ab, wäscht und trocknet bei 50 °C. Man isoliert 25 Teile der Verbindung der Formel

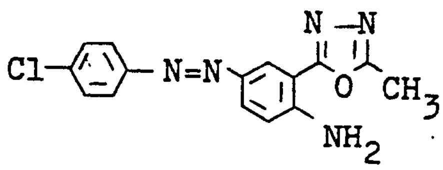

mit Schmelzpunkt 220 - 223 °C.

Analog

zu den beschriebenen Methoden können auch die in den folgenden Tabellen gekennzeichneten Verbindungen hergestellt werden.

Tabelle 1

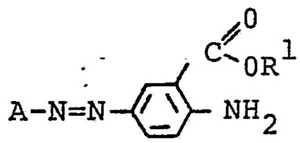

| Bsp. | A | $R^1$ | Schmp. ($^oC$) | λ max. (nm) |
|---|---|---|---|---|
| 14 | 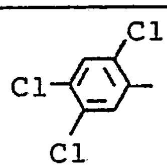 | $CH_3$ | 208-210 | 413 |
| 15 | " | 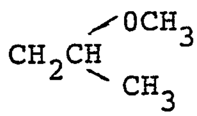 | 126-128 | |
| 16 | 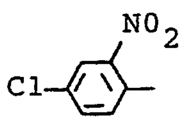 | $CH_3$ | 185-187 | |
| 17 | 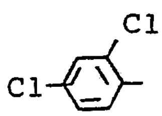 | 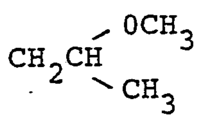 | 103-105 | |
| 18 | 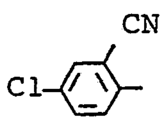 | $C_{10}H_{21}$ | 49 | |
| 19 | 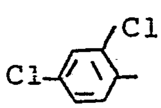 | $CH_2CH_2O$- 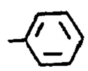 | 139-141 | |
| 20 | 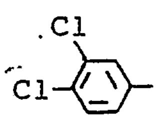 | 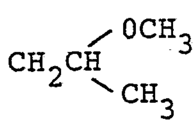 | 101-104 | |
| 21 | 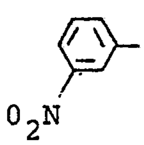 | 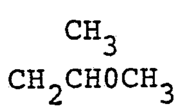 | 137-140 | |
| 22 | 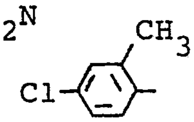 | $(CH_2)_3O(CH_2CH_2O)_2CH_3$ | | 364 |

| Bsp. | A | $R^1$ | Schmelzp. (°C) | λmax. (nm) |
|---|---|---|---|---|
| 23 | 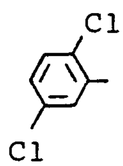 | $C_6H_{13}$(n) | 127 - 131 | |
| 24 | " | $C_8H_{17}$(i) | 88 - 92 | |
| 25 | " | $CH_2\overset{OCH_3}{C}HCH_3$ | 121 - 124 | 409 |
| 26 | 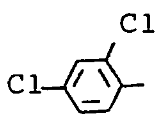 | $C_8H_{17}$(i) | | 393 |
| 27 | 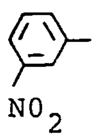 | $CH_3$ | | 396 |
| 28 | " | $C_3H_7$(n) | 160 - 163 | 358, 398 |
| 29 | 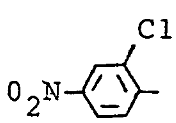 | " | | 433 |
| 30 | 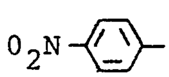 | $CH_3$ | 232 | 424 |
| 31 | 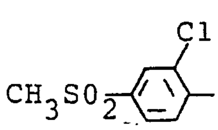 | $C_6H_{13}$(n) | | 416 |
| 32 | " | $CH_3$ | | 415 |

| Bsp. | A | $R^1$ | Schmp. (°C) | λmax. (nm) |
|---|---|---|---|---|
| 33 | $CH_2=CH-CH_2SO_2$-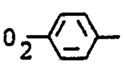 | $CH_3$ | 136-138 | 360 Schulter, 403 |
| 34 | 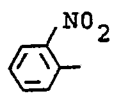 | $CH_3$ | 195-197 | 356 Schulter, 402 |
| 35 | 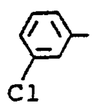 | OH<br>$CH_2CH$<br>$CH_3$ | | 391, Schulter 361 |
| 36 | 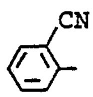 | $CH_3$ | 210-213 | 363; 407,5 |
| 37 | " | $C_2H_5$ | 158-160 | |
| 38 | " | $C_3H_7$(n) | 128-130 | |
| 39 | 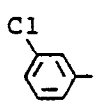 | $OCH_3$<br>\|<br>$CH_2CHCH_3$ | 97 - 99 | |
| 40 | 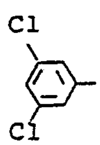 | " | 104-107 | |
| 41 | 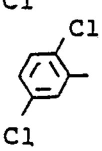 | $CH_2)_3OCH_2$-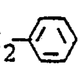 | | 408 |
| 42 | " | $(CH_2)_3O(CH_2)_2O$-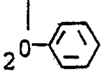 | | 408 |

| Bsp. | A | R | Schmp. (°C) | λmax. (nm) |
|---|---|---|---|---|
| 43 | 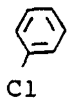 | 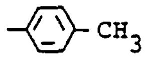 | | 380 |
| 44 | " | 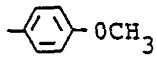 | | 382 |
| 45 | " | 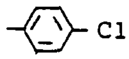 | | 379 |
| 46 | " | $CH_2CH=CH_2$ | | 391 |
| 47 | " | 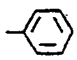 | | 376 |
| 48 | 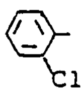 | 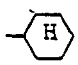 | 102-106 | 392 |
| 49 | " | $C_3H_{13}$(n) | 77 | 355, 393 |
| 50 | 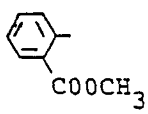 | $(CH_2)_3OC_2H_5$ | 52 | |
| 51 | " | $(CH_2)_3O(CH_2)_2OCH_3$ | 30 | |

| Bsp. | A | $R^1$ | Schmp. (oC) | λmax. (nm) |
|---|---|---|---|---|
| 52 | 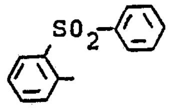 | $(CH_2)_3O(CH_2CH_2O)_2C_4H_9(n)$ | flüssig | |
| 53 | " | $CH_2CH_2$- 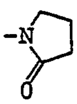 | 85 | |

Verwendet man bei der Herstellung der in Tabelle 1 beschriebenen Verbindungen anstelle der dort eingesetzten Amine cyclische Amine, so erhält man die entsprechenden Amide wie zum Beispiel

Schmp. (°C)

Bsp. 54 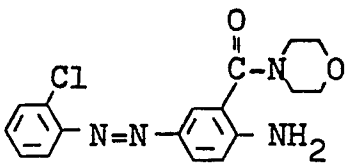 154 - 157

Bsp. 55 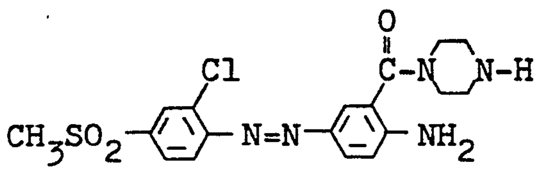

Bsp. 56 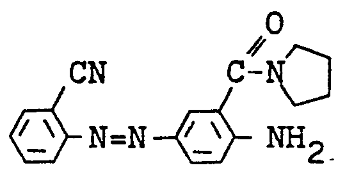

Bsp. 57 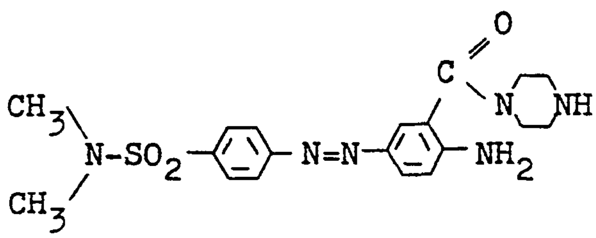

Bsp. 58 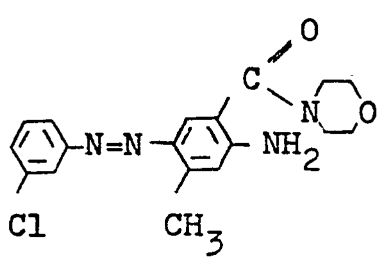 163-166

Analog zu den in den Beispielen 1, 2, 5 und 9 benannten Verfahren lassen sich z.B. folgende Azoanthranilsäurederivate herstellen:

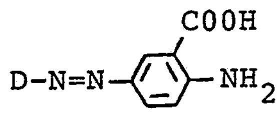

| D | Schmelzpunkt ($^{o}C$) | λmax. (nm) |
|---|---|---|
| 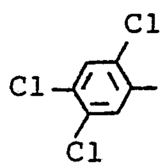 | | 420 |
| 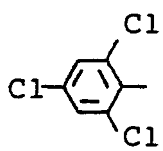 | | 375 |
| 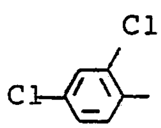 | 200 - 202 | 412 |
| 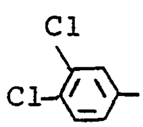 | | 401 |
| 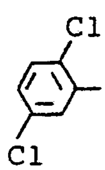 | | 416 |
| 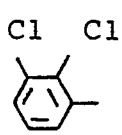 | 255 - 256 | 406 |

| D | Schmelzpunkt ($^{o}C$) | $\lambda$max. (nm) |
|---|---|---|
| 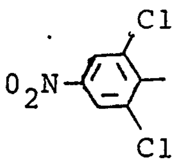 | 230 - 235 | 394 |
| 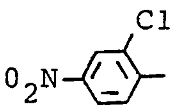 | | 441 |
| 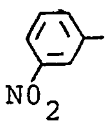 | | 420 |
| 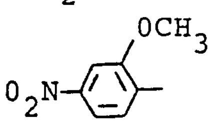 | | 409 |
| 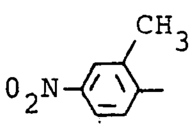 | | 433 |
| 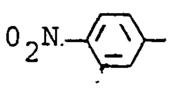 | 232 | 424 |
| 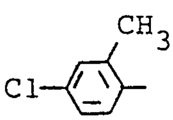 | | 397 |
| 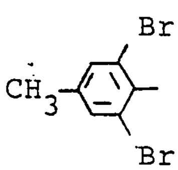 | 180 | |
| 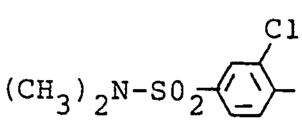 | | 410 (Isobutanol) |

| D | Schmelzpunkt (°C) | $\lambda$max. (DMF) |
|---|---|---|
| 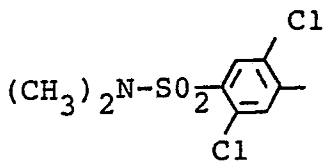 | | 495 |
| 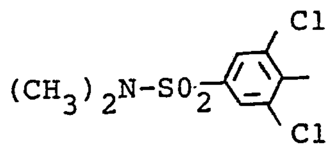 | | 943 |
| 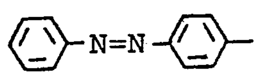 | | 425 |
| 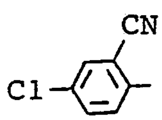 | | 475 |
| 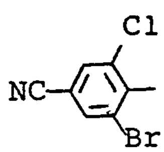 | | 391 |
| 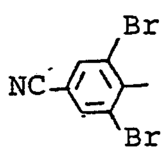 | 249 - 253 | |
| 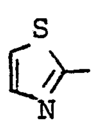 | 164 - 170 | |
| 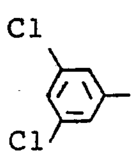 | | 412 |

| D | Schmelzpunkt ($^{o}C$) | λ max. (DMF) |
|---|---|---|
| 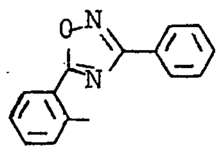 | | 353 |
| 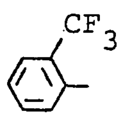 | | |
| 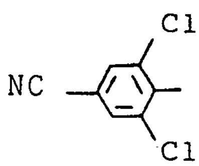 | | |
| 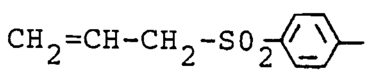 | | |
| 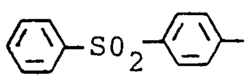 | | |
| 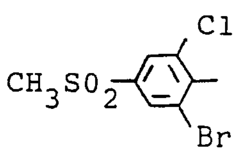 | | |
| 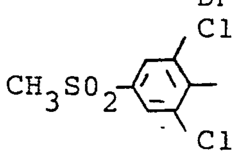 | | |
| 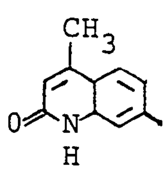 | | |

| D | Schmelzpunkt ($^oC$) | λ max. (nm) |
|---|---|---|
| 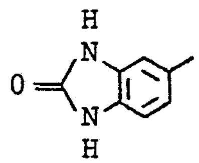 | | |
| 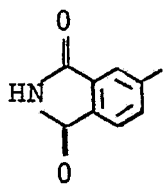 | | |
| 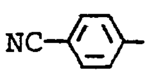 | | |
| 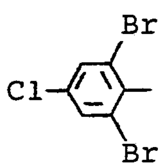 | | |
| 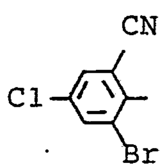 | | |
| 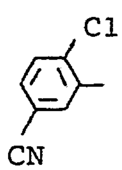 | | |

Tabelle 2

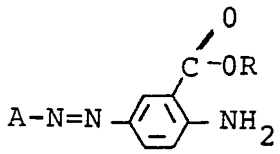

| Bsp. | A | R | Schmp. (°C) | λmax. (nm) |
|---|---|---|---|---|
| 59 | 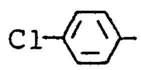 | $CH_2CH_2CH(OCH_3)CH_3$ | 103-104 | |
| 60 | " | $C_6H_{13}$(n) | 106-107 | |
| 61 | 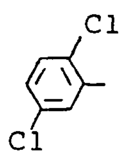 | $C_6H_5$ | 187-188 | 405 |
| 62 | 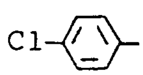 | $C_{10}H_{21}$ | 67-70 | |
| 63 | 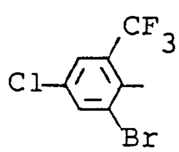 | $CH(CH_3)_2$ | 113-17 | |
| 64 | 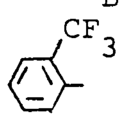 | $CH_3$ | 104-106 | |
| 65 | " | $C_2H_5$ | 97 - 99 | |
| 66 | " | $C_4H_9$(n) | 75 - 77 | |

| Bsp. | A | R | Schmp. ($^oC$) | λmax. (nm) |
|---|---|---|---|---|
| 67 | 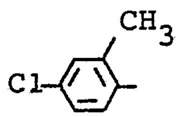 | $CH_3$ | (160-170 Sintern) 190-195 | |
| 68 | 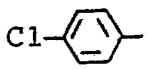 | $C_6H_{13}$(i) | | |
| 69 | " | $C_7H_{15}$(n) | | |
| 70 | " | $C_7H_{15}$(i) | | |
| 71 | " | $C_8H_{17}$(n) | | |
| 72 | " | $CH_2CH-C_4H_9$(n)<br>\|<br>$C_2H_5$ | | |
| 73 | " | | | |
| 74 | " | $C_9H_{19}$(n) | | |
| 75 | 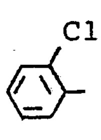 | $C_{10}H_{21}$(n) | 65-68 | |
| 76 | 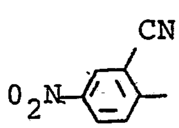 | $CH_3$ | 228 Zers. | 461 |

| Bsp. | A | R | Schmp. (°C) | λ max. (nm) |
|---|---|---|---|---|
| 77 | 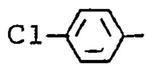 | $C_6H_5$ | 188-192 | 375 |
| 78 | " | $CH_2-C_6H_6$ | | |
| 79 | " | $CH_2CH_2C_6H_5$ | | |
| 80 | " | $(CH_2CH_2O)_2CH_3$ | 62-64 | Schulter 365<br>389 |
| 81 | " | $(CH_2CH_2O)_2C_2H_5$ | 58-60 | |
| 82 | " | $(CH_2CH_2O)_2C_4H_9$(n) | | |
| 83 | " | $(CH_2CH_2O)_3CH_3$ | | |
| 84 | " | $(CH_2CH_2O)_3C_4H_9$(n) | | |
| 85 | " | $(CH_2CH_2O)_4C_4H_9$(n) | | |
| 86 | " | 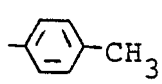 | 152-154 | |

| Bsp. | A | R | Schmp. (°C) | λ max. (nm) |
|---|---|---|---|---|
| 87 | 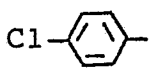 | 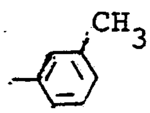 | | |
| 88 | " | 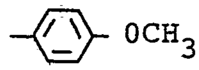 | | |
| 89 | " | 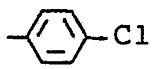 | 205-210 | |
| 90 | " | 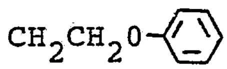 | | |
| 91 | " | $CH_2CH_2NHCOCH_3$ | | |
| 92 | " | $CH_2CH_2CH_2NHCOCH_3$ | | |
| 93 | " | 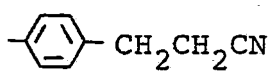 | | |
| 94 | " | $CH_2CH_2OH$ | | |
| 95 | " | 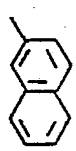 | | |

Verwendet man anstelle der in Tabelle 2 angegebenen Reste A z. B. die folgenden Reste:

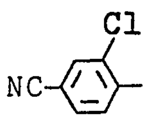 , 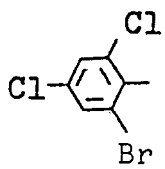 , 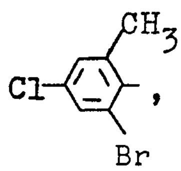 , 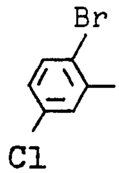 , 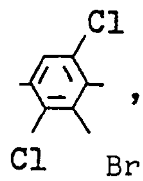 ,

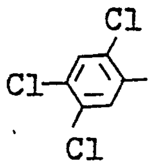 , 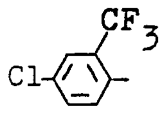 , 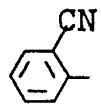 , 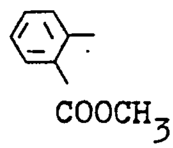 ,

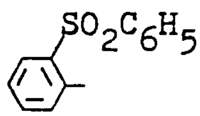 , 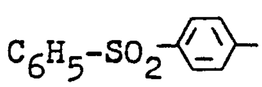 , 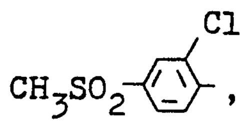 , 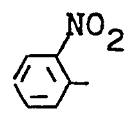 ,

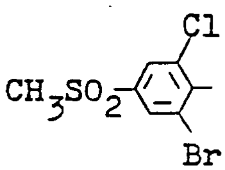 , 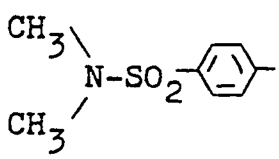 , 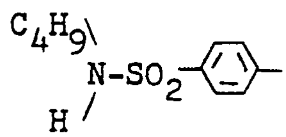 ,

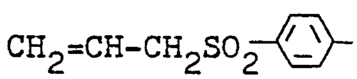 , 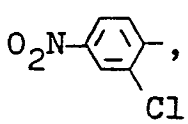 , 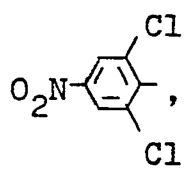 , 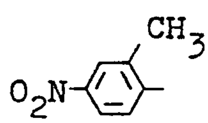 ,

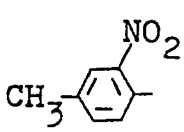 , 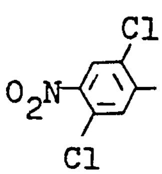 , 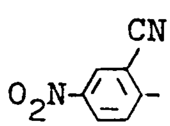 , 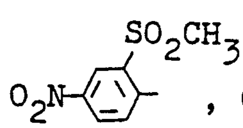 , 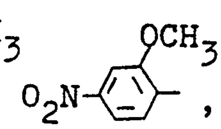 ,

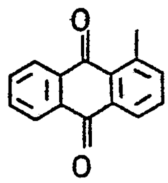 , 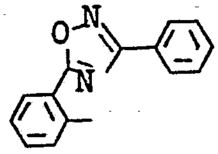 , 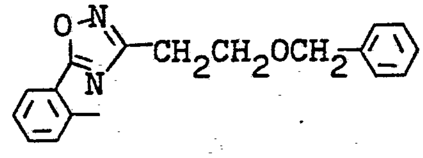 ,

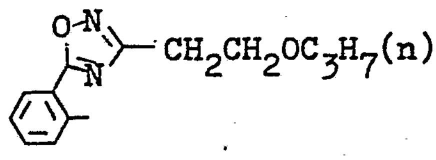 , 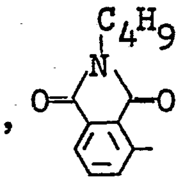 , 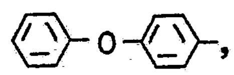 ,

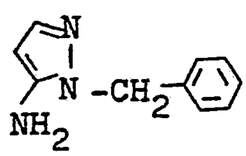 , 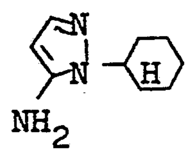 , 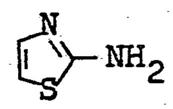 , 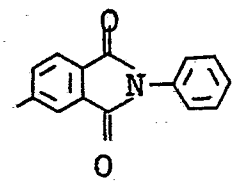 ,

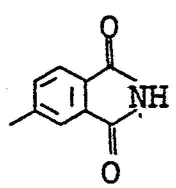 , 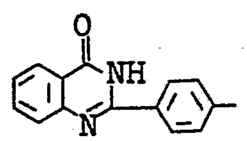 , 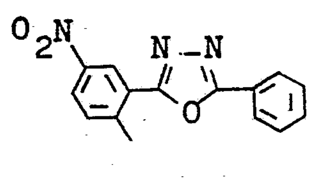 ,

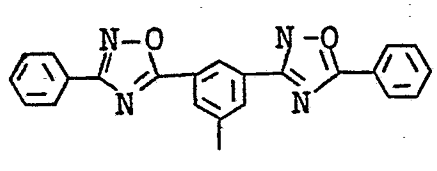 , 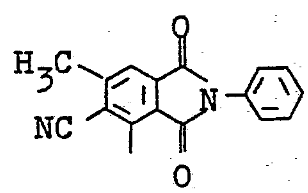 ,

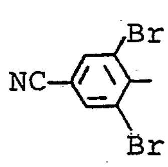 , 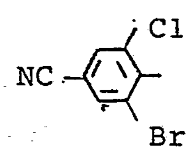 , 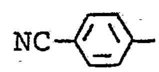 , 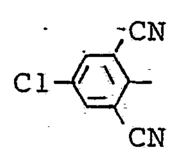

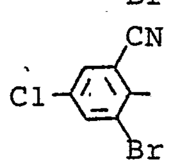 , 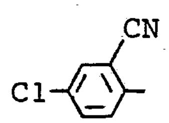 , 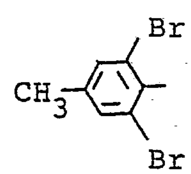

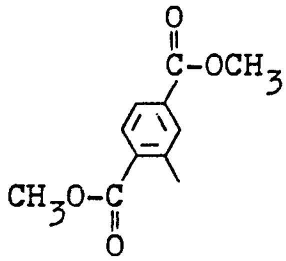 , 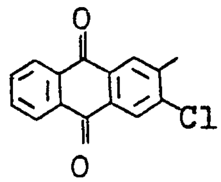 , 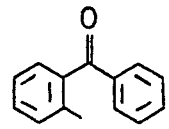 ,

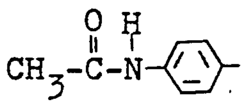 , 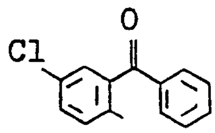 , 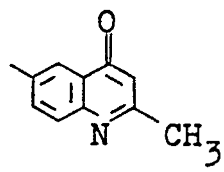 ,

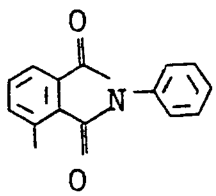 oder 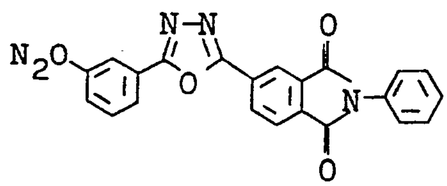

so erhält man die entsprechenden Diazokomponenten gemäß der in den Beispielen beschriebenen Arbeitsweise.

Tabelle 3

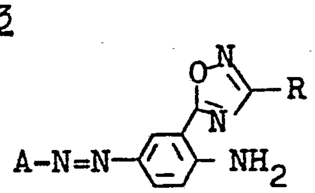

| Bsp. | A | R | Schmp. ($^0C$) | λ max. (nm) |
|---|---|---|---|---|
| 87 | 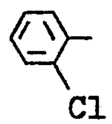 | $CH_3$ | | 360 |
| 88 | " | $C_2H_5$ | | 362 |
| 89 | " | $C_6H_{13}$ | | |
| 90 | " | $CH_2OCH_3$ | | 359 |
| 91 | " | $CH_2CH_2OCH_3$ | | |
| 92 | " | $(CH_2)_2OC_2H_5$ | | |
| 93 | " | $(CH_2)_2OC_3H_7$(n) | | 362 |
| 94 | " | $(CH_2)_2OC_3H_7$(i) | | 362 |

| Bsp. | A | R | Schmp. (°C) | λ max. (nm) |
|---|---|---|---|---|
| 95 | 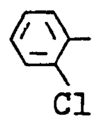 | $(CH_2)_2O(CH_2)_2OCH_3$ | | 360 |
| 96 | " | $(CH_2CH_2O)_3CH_3$ | | |
| 97 | " | $(CH_2CH_2O)_2C_2H_5$ | | |
| 98 | " | $(CH_2CH_2O)_2C_4H_9(n)$ | | |
| 99 | " | $(CH_2CH_2O)_3C_4H_9(n)$ | | |
| 100 | " | 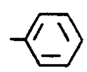 | | 400 |
| 101 | " | 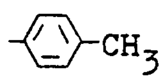 | | |
| 102 | " | 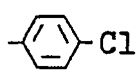 | | 398 |
| 103 | " | 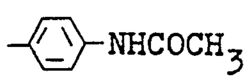 | | |
| 104 | " | $CH_2CH_2SO_3H$ | | |

| Bsp. | A | R | Schmp. (°C) | λ max. (nm) |
|---|---|---|---|---|
| 105 | 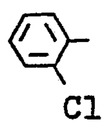 | $CH_2SO_3H$ | | 353 |
| 106 | " | 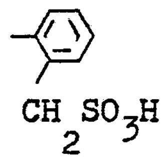 | | 373 |
| 107 | " | 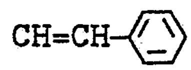 | | |
| 108 | " | 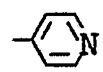 | | |
| 109 | " | 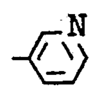 | | |
| 110 | " | 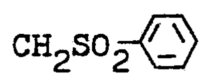 | | |
| 111 | " | $CH_2-C_6H_5$ | | |
| 112 | 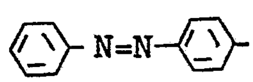 | $CH_3$ | | |
| 113 | " | $(CH_2)_2OC_3H_7$ | | |

| Bsp. | A | R | Schmp. (°C) | max. λ (nm) |
|---|---|---|---|---|
| 114 | 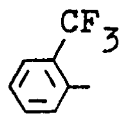 | $(CH_2)_2OC_3H_7$ | | 368 |
| 115 | $CH_2=CH-CH_2-SO_2-$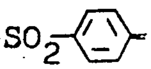 | " | | |
| 116 | $CH_3SO_2-$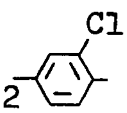 | " | | |
| 117 | $C_4H_9(n)-SO_2-$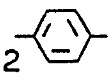 | " | | |
| 118 | 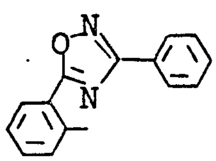 | $C_6H_5$ | | |

Tabelle 4

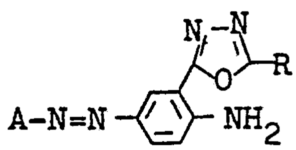

| Bsp. | A | R | Schmp. (°C) | λ max. (nm) |
|---|---|---|---|---|
| 119 | 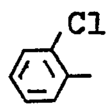 | $CH_3$ | | |
| 120 | " | $C_2H_5$ | | |
| 121 | " | $C_3H_7$ | | 364, 420 |
| 122 | " | $CH_2OCH_3$ | | 360, 418 |
| 123 | " | $CH_2OC_6H_5$ | | |
| 124 | " | $C_6H_5$ | | |
| 125 | 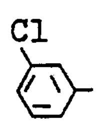 | $CH_3$ | | |
| 126 | " | $C_2H_5$ | | |

| Bsp. | A | R | Schmp. (°C) | λ max. (nm) |
|---|---|---|---|---|
| 127 | 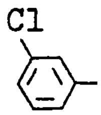 | $C_3H_7$ | | |
| 128 | " | $CH_2OCH_3$ | | |
| 129 | " | $C_6H_5$ | | |
| 130 | 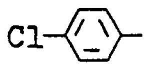 | $C_2H_5$ | | |
| 131 | " | $C_3H_7$ | | |
| 132 | " | $CH_2OCH_3$ | | |
| 133 | " | $C_6H_5$ | | |
| 134 | 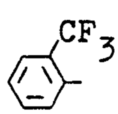 | " | | |
| 135 | 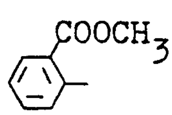 | " | 250-254 | |
| 136 | 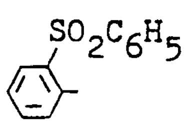 | " | | |

Beispiel 137

11,0 Teile der Verbindung der Formel

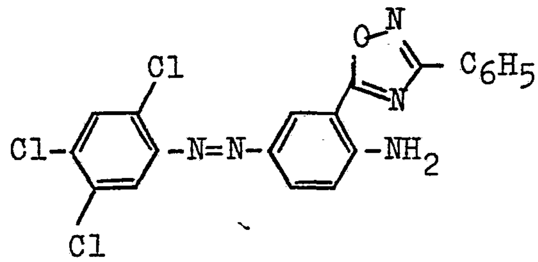

werden bei 40°C mit 200 Raumteilen Wasser, 5 Raumteilen 16 %iger Salzsäure und 0,5 Teilen eines Netzmittels 1 Stunde verrührt. Dann tropft man innerhalb von 2 Stunden bei 40 bis 60°C 6,4 Teile Brom zu, rührt 2 Stunden bei 80°C nach und kühlt auf Raumtemperatur ab. Nach dem Verdünnen mit 200 Raumteilen Wasser, Abfiltrieren, Waschen mit Wasser und Trocknen erhält man 13 Teile der Verbindung der Formel

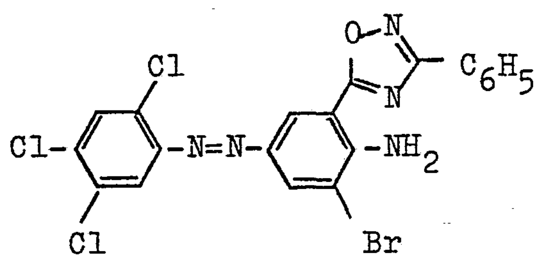

mit einem Schmelzpunkt von 230 bis 235°C.

Beispiel 138

Bromiert man analog zu der in Beispiel 137 beschriebenen Arbeitsweise 15,0 Teile der Aminoazoverbindung der Formel

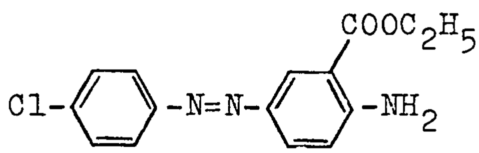

mit 8,8 Teilen Brom, so erhält man 18 Teile der Diazokomponente der Formel

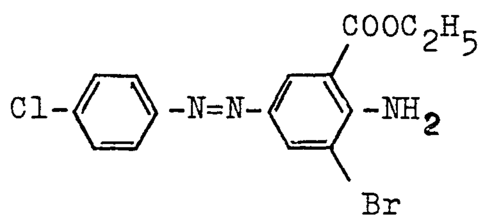

mit einem Schmelzpunkt von 133 bis 135°C.

Durch Chlorieren erhält man analog Beispiel 137 die entsprechenden Chlorverbindungen.

Tabelle

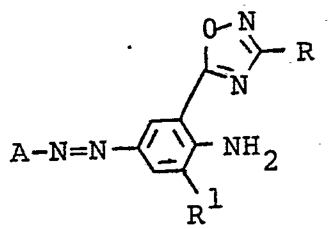

| Bsp. | A | $R^1$ | R | Schmp. ($^oC$) | λmax. (nm) |
|---|---|---|---|---|---|
| | 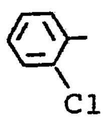 | H | $CH_2CH_2OCH_2$ | 102-104 | 361 |
| | " | Br | $C_6H_5$ | | 373 |
| | 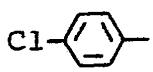 | " | " | | 372 |
| | " | H | 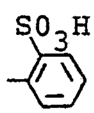 | | 373 |
| | 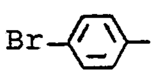 | H | " | | 385 |
| | 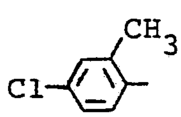 | H | " | 194 | 350 |
| | " | Cl | " | | 351 |
| | 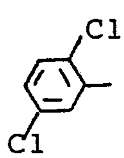 | " | " | | 412 |
| | " | Br | " | | 380 |

| Bsp. | A | $R^1$ | R | Schmp. (°C) | max. (nm) |
|---|---|---|---|---|---|
| | 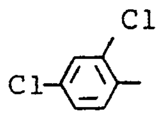 | H | 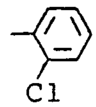 | | 376, 403 |
| | " | H | 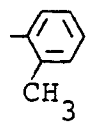 | | 397 |
| | 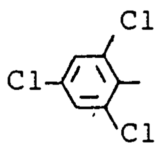 | H | $C_6H_5$ | | 370 |
| | 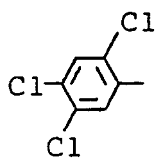 | H | " | 249-251 | 362, 419 |
| | 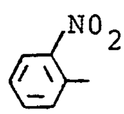 | Br | " | | 376 |
| | 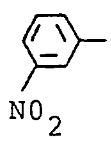 | H | " | 246-290 | |
| | 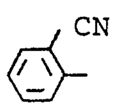 | H | " | Zers. > 220 | 373 |
| | 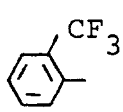 | H | " | 176-179 | |
| | 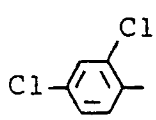 | H | " | 246-249 | |
| | 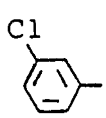 | H | " | 246-249 | |

| Bsp. | A | $R^1$ | R | Schmp. (°C) | max. (nm) |
|---|---|---|---|---|---|
| | 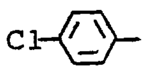 | H | $C_6H_5$ | 240-241 | |
| | 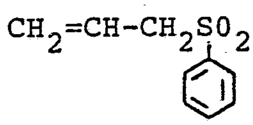 | H | " | | |
| | 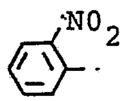 | Br | " | 137-140 | |
| | 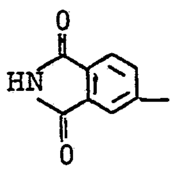 | H | " | 263-265 | |
| | 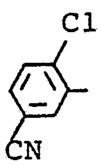 | | | | |

Tabelle

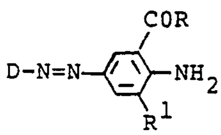

| Bsp. | D | R | $R^1$ | Schmelzpunkt (°C) | λmax. (nm) |
|---|---|---|---|---|---|
| | 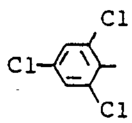 | $OC_2H_4OCH_3$ | Br | 128 - 131 | 361 |
| | 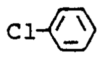 | 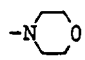 | H | 188 | 398 |
| | " | 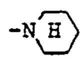 | H | 160 - 164 | |
| | " | $OCH_3$ | Br | | 356,5 |
| | 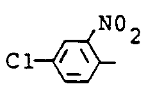 | " | " | 189 - 192 | 405<br>355 Schulter |
| | 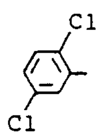 | " | " | | 357; 403 |
| | 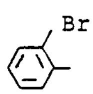 | $OCH_3$ | H | | 353; 400 |
| | " | $OC_2H_5$ | H | | 354; 397 |

| Bsp. | D | R | $R^1$ | Schmelzpunkt (°C) | max. (nm) |
|---|---|---|---|---|---|
| | 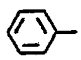 | $OC_3H_7$ | H | | 353; 396 |
| | 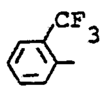 | $OC_6H_{13}$(n) | H | 50 - 52 | |
| | 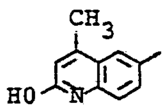 | $OCH_3$ | H | | |
| | 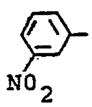 | $OC_4H_9$ | H | 139 - 141 | 355; 396,5 |
| | " | $OCH_2CHCH_3$<br>$OCH_3$ | H | 137 - 140 | |
| | 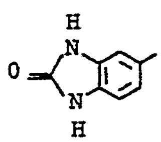 | $OCH_3$ | H | 221-225 | |

Tabelle

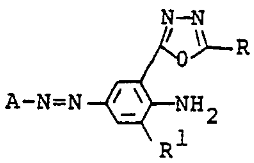

| Bsp. | A | $R^1$ | R | Schmp. (°C) | λmax. (nm) |
|---|---|---|---|---|---|
| | 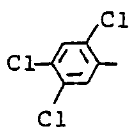 | H | $CH_3$ | 202 - 205 | |
| | " | H | $C_6H_5$ | | 361, 420 |
| | " | H | $CH_3$ | 181 - 185 (Zers.) | |
| | 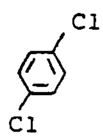 | H | " | | |
| | " | Br | $C_6H_5$ | | |
| | " | H | $C_6H_5$ | | |
| | 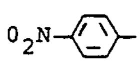 | " | " | | |
| | 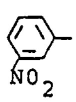 | " | " | | |
| | 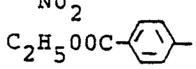 | " | " | | |

Beispiel 139

33 Teile der Diazokomponente der Formel

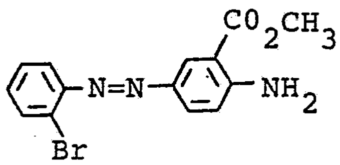

werden bei 80 - 100 °C in 300 Teilen Dimethylformamid mit 10 Teilen CuCN versetzt. Man rührt 1 Stunde bei 120 - 130 °C, kühlt auf 20 °C ab und läßt 65 Teile wäßrige, ca. 25 %ige Ammoniaklösung zulaufen. Nach 30 Minuten Nachrührzeit tropft man noch 100 Teile Wasser hinzu, läßt die Fällung noch 30 Minuten nachrühren und saugt dann ab. Zur weiteren Reinigung rührt man das Nutschgut nochmals mit 100 Teilen Methanol an, filtriert ab und trocknet bei 80 °C. Man isoliert 37 Teile der gelben Verbindung der Formel

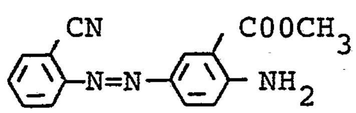

mit einem Schmelzpunkt von 208 - 210 °C.

Beispiel 140

40 Teile der Verbindung der Formel

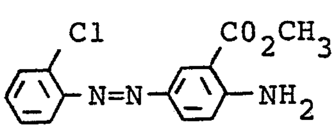

(Schmelzpunkt 138 - 140 °C)

werden unter Rühren bei 15 - 25 °C in Portionen in 105 Teile Oleum, 23 %ig, gegeben. Anschließend rührt man das Gemisch 6 Stunden bei 40 °C. Nach Abkühlen auf Raumtemperatur wird die Sulfierung auf 400 Teile Eis ausgerührt. Die erhaltene Fällung wird mit Natronlauge oder Natriumacetat auf pH 4 bis 6 abgestumpft. Das ausgefallene Produkt der Formel

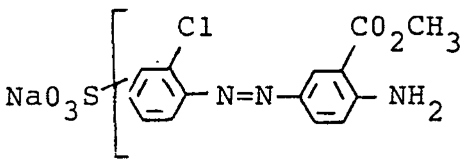

wird abgesaugt, mit etwas Wasser gewaschen und getrocknet. Man erhält 42 Teile eines gelbbraunen Pulvers, das sich in Wasser mit gelber Farbe löst. Das UV-Absorptionsspektrum, aufgenommen mit dem Bittman Spektralphotometer ACTA III, zeigt ein Maximum bei 394 nm (pH 5, in Wasser). Die Diazokomponente ist dünnschichtchromatographisch einheitlich und zersetzt sich beim Erhitzen bei Temperaturen oberhalb von 270 °C.

Analog zu der in Beispiel 139 beschriebenen Verfahrensweise können die in der folgenden Tabelle genannten Verbindungen synthetisiert werden. Der Austausch von Brom gegen Cyan erfolgt dabei in der Regel sehr leicht. Beim Austausch von Chlor gegen Cyan, der in der Regel schwerer erfolgt, wählte man anstelle von Dimethylformamid N-Methylpyrrolidon und erhöhte die Reaktionstemperatur auf bis zu 170 - 180 °C.

Tabelle

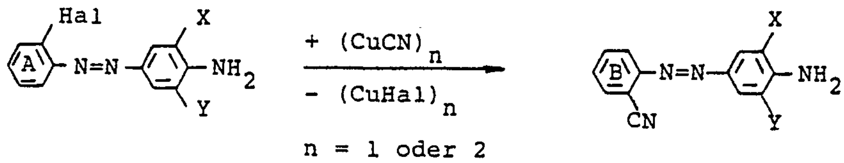


| Bsp. | 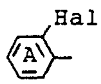 | 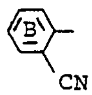 | X | Y | Schmp. (°C) | λmax. (nm) |
|---|---|---|---|---|---|---|
| | 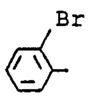 | 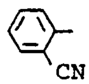 | $CO_2C_2H_5$ | H | 158-160 | |
| | " | " | $CO_2C_3H_7$ | H | 128-130 | 351, 409 |
| | " | " | $CO_2C_6H_5$ | H | | |
| | " | " | $CO_2C_6H_{13}(n)$ | H | | |
| | " | " | 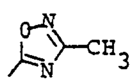 | H | | |
| | " | " | 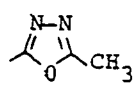 | H | | 373 |
| | " | " | 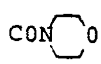 | H | | |
| | 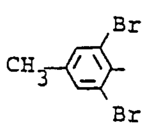 | 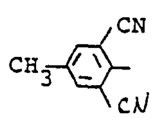 | " | H | | |

| Nr. | 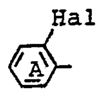 | 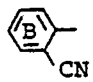 | X | Y | Schmp. (°C) | λmax. (nm) |
|---|---|---|---|---|---|---|
| | 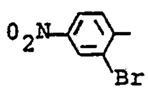 | 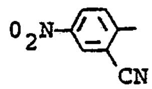 | $COOC_2H_5$ | H | | 462 |
| | " | " | $COOC_4H_9$ | H | | |
| | " | " | 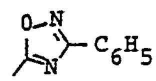 | H | | |
| | " | " | $COOCH_3$ | Br | | |
| | " | " | 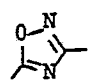 | Br | | |
| | 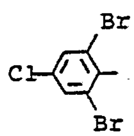 | 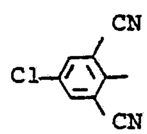 | " | Br | | |
| | 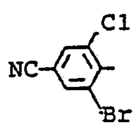 | 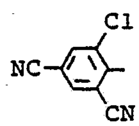 | " | H | | |
| | " | " | " | Br | | |
| | " | " | " | Cl | | |
| | 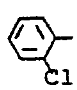 | 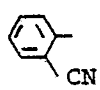 | $CO_2CH_3$ | H | 208-210 | |
| | " | " | $CO_2C_2H_5$ | Cl | | |

| Bsp. | 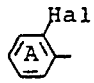 | 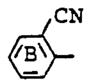 | X | Y | Schmp. (°C) | max. (nm) |
|---|---|---|---|---|---|---|
| | 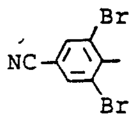 | 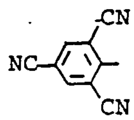 | 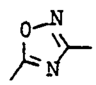 $C_6H_5$ | H | | |
| | " | " | $CO_2C_6H_{13}$(n) | " | | |
| | " | " | $CO_2C_8H_{17}$(i) | " | | |

Analog zu der in den Beispielen 1, 2, 5, 9 beschriebenen Arbeitsweise lassen sich z.B. auch folgende Azo-Isatosäure-anhydride herstellen:

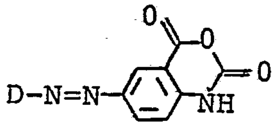

| D | Schmp. | λmax. (nm) in Dimethylformamid |
|---|---|---|
| 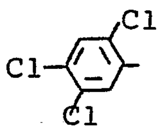 | 320 - 324 | 457 |
| 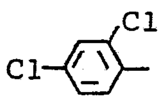 | 305 (Zers.) | 360, 418 (zwei Maxima) |
| 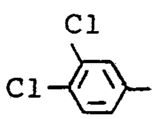 | 268 - 271 | |
| 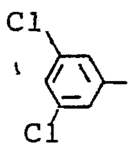 | 286 - 291 | |
| 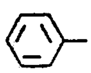 | | 391,5 |
| 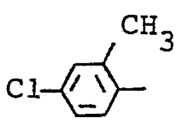 | | 355, 419 |
| 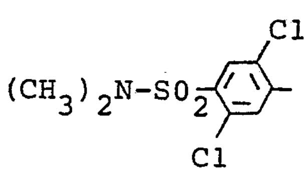 | 163 - 169 | 375, 474 |
| 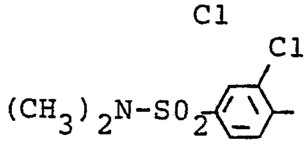 | 176 - 180 | |

| D | Schmp. (°C) | λmax. (nm) | in Dimethylformamid |
|---|---|---|---|
| 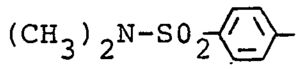 | 275 - 280 | | |
| 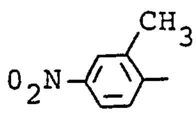 | 268 - 270 | 369, 460 | |
| 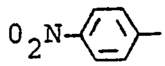 | | 366, 465 | |
| 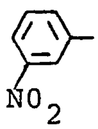 | 301 - 304 | | |
| 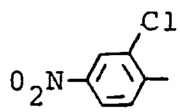 | | 375, 484 | |
| 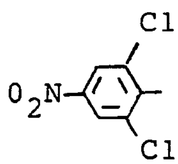 | 280 - 284 | | |
| 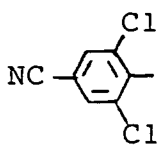 | | | |
| 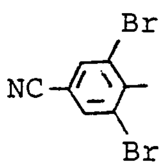 | | | |
| 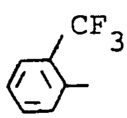 | 278 - 284 | | |
| 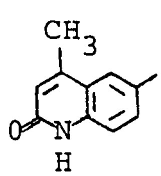 | | | |

| D | Schmp. (°C) | λmax. (nm) |
|---|---|---|
| 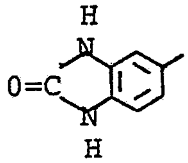 | | |
| 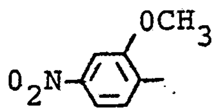 | 295 - 299 | |
| 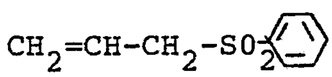 | 265 - 270 | |
| 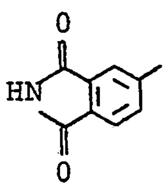 | | |
| 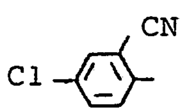 | 265 (Zers.) | |
| 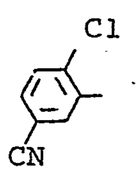 | | |
| 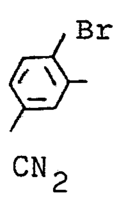 | | |

| D | Schmp. ($^{o}C$) | $\lambda$ max. (nm) |
|---|---|---|
| 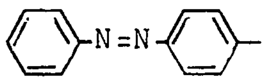 | | |
| 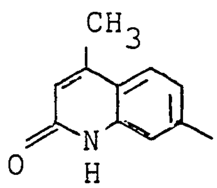 | | |
| 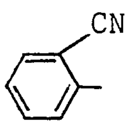 | | |

Patentansprüche

1. Aminoazoverbindungen der allgemeinen Formel

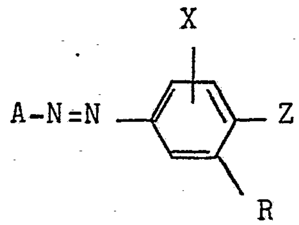

in der

A der Rest einer Diazo- oder Kupplungskomponente und

R ein Rest der Formel

CN, COOR$^1$, COSR$^1$, CONH$_2$, CONHNH$_2$, CONH-NH-CO"$^1$,

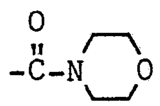 , 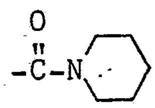 , 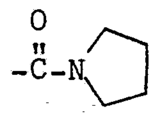 ,

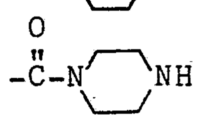 , 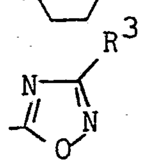 , 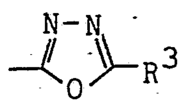 oder 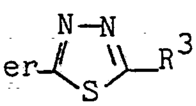

Z Amino,

R und Z zusammen ein Rest der Formel -NHCO-O-CO-,

X Wasserstoff, Chlor, Brom, Methoxy, Äthoxy, Propoxy, $SO_3H$, Butoxy, Phenoxy, Methyl, Äthyl, Propyl, Butyl, Acetylamino, Dimethylamino, Diäthylamino, Carboxyl oder $-NO_2$ sind, wobei

$R^1$ gegebenenfalls substituiertes Alkyl, Alkenyl, Cycloalkyl oder Aryl,

$R^2$ Wasserstoff oder gegebenenfalls substituiertes Alkyl und

$R^3$ Wasserstoff oder einen Rest $R^1$ bedeuten und wobei Aminobenzoesäureamide, Mono- und Dichloraniline als Diazokomponenten A ausgeschlossen sind, wenn $R^1$ $C_1$- bis $C_4$-Alkyl, Methoxyäthyl oder Butoxyäthoxyäthyl ist.

2. Verbindungen gemäß Anspruch 1 der Formel

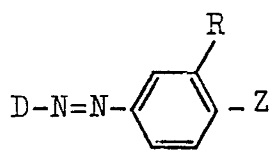
,

in der

D der Rest einer Diazokomponente ist und Z und

R die angegebene Bedeutung haben.

3. Verwendung der Verbindungen gemäß Anspruch 1 oder 2 als Diazokomponenten.